# EUROPÄISCHE PATENTSCHRIFT

(11) **EP 2 776 001 B1**
(45) Veröffentlichungstag und Bekanntmachung des Hinweises auf die Patenterteilung: **14.06.2017**
(21) Anmeldenummer: 12781097.6
(22) Anmeldetag: 05.11.2012
(51) Int. Cl.: A61K 8/49, A61Q 5/06, C09B 62/82

(54) **ANIONISCHE AZOFARBSTOFFE ZUM FÄRBEN VON KERATINHALTIGEN FASERN**
ANIONIC AZO DYESTUFFS FOR COLOURING KERATIN-CONTAINING FIBRES
COLORANTS AZOÏQUES ANIONIQUES SERVANT A COLORER DES FIBRES KÉRATINIQUES

(30) Priorität: 08.11.2011 DE 102011085907; 24.02.2012 DE 102012202818
(43) Veröffentlichungstag der Anmeldung: 17.09.2014
(73) Patentinhaber: Henkel AG & Co. KGaA, 40589 Düsseldorf (DE)
(72) Erfinder: GROß, Wibke, 41836 Hückelhoven (DE); NEMITZ, Ralph, 41363 Jüchen (DE); MOCH, Melanie, 41542 Dormagen (DE); KROOS, Astrid, 40789 Monheim (DE); GEBERT, Antje, 40545 Düsseldorf (DE)
(86) Internationale Anmeldenummer: PCT/EP2012/071814
(87) Internationale Veröffentlichungsnummer: WO 2013/068311

(56) Entgegenhaltungen:
- EP-A1- 1 915 984
- DE-A1- 2 317 133
- SHIBUSAWA, TAKAO ET AL: "Dyeing properties of disperse dyes. VI. Interactions between .beta.-cyclodextrin and 4-aminoazobenzene derivatives and the effect of the interactions on the dyeing properties of 4-aminoazobenzene derivatives", NIPPON KAGAKU KAISHI , (12), 2171-7 CODEN: NKAKB8; ISSN: 0369-4577, 1975, XP9166395,

## Beschreibung

Die Anmeldung betrifft Azofarbstoffe in Mitteln zum Färben und gegebenenfalls gleichzeitigen Aufhellen von keratinhaltigen Fasern, insbesondere menschlichen Haaren.

Für das Färben von keratinischen Fasern kommen im allgemeinen entweder direktziehende Farbstoffe oder Oxidationsfarbstoffe zur Anwendung. Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Des Weiteren können einige Oxidationsfarbstoffvorprodukte bzw. bestimmte Mischungen von Oxidationsfarbstoffvorprodukten bei Personen mit empfindlicher Haut sensibilisierend wirken. Direktziehende Farbstoffe werden unter schonenderen Bedingungen appliziert. Ihr Nachteil liegt jedoch darin, dass die Färbungen häufig nur über unzureichende Echtheitseigenschaften verfügen, insbesondere bei der Haarwäsche, aber auch gegenüber äußeren Einflüssen, wie Sonnenlicht oder reaktiven Umweltchemikalien, wie beispielsweise Schwimmbadwasser. Direktziehende Farbstoffe werden häufig auch zur Nuancierung von oxidativen Färbungen eingesetzt.

Eine besondere Herausforderung für die Haarfärbung mit direktziehenden Farbstoffen stellt die gleichmäßige Färbung von häufig vorbehandeltem Haar, wie beispielsweise gebleichtem oder dauergewelltem Haar, dar, bei dem die Faser in den verschiedenen Längen oder verschieden behandelten Bereichen eine sehr unterschiedlich starke Vorschädigung besitzt. Während der Färbung selbst kann das Färbemittel auf unterschiedlich vorgeschädigtem Haar ein ungleichmäßiges Färbeverhalten zeigen, aber auch durch wiederholte Haarwäsche können die Farbstoffe in den unterschiedlichen Haarbereichen verschieden stark auswaschen werden, wodurch ein uneinheitliches und damit unerwünschtes Farbergebnis erzielt wird.

Für die starke Aufhellung von dunklen Haaren wird nicht nur Wasserstoffperoxid allein, sondern eine Kombination aus Wasserstoffperoxid und Persulfaten (z.B. Ammoniumpersulfat, Kaliumpersulfat und/oder Natriumpersulfat) eingesetzt. Soll also dunkles Haar in einem Schritt stark aufgehellt und gleichzeitig in einem leuchtenden Farbton gefärbt werden, ist der Einsatz einer Mischung aus Wasserstoffperoxid, Persulfaten und einem direktziehenden Farbstoff von Vorteil. Obwohl dem Fachmann zum Färben von Haaren viele intensiv färbende direktziehende Farbstoffe bekannt sind, so kennt er doch nur eine sehr begrenzte Auswahl, die die starken oxidativen Bedingungen, wie sie eine Mischung der oben genannten Oxidationsmittel darstellt, ohne Zersetzung überstehen. Zudem weisen die aus dem Stand der Technik bekannten oxidationsstabilen Farbstoffe im Hinblick auf ihre übrigen Echtheitseigenschaften gravierende Nachteile auf.

Für das gleichzeitige Färben und starke Aufhellen von Haaren besteht damit nach wie vor ein Bedarf an Farbstoffen mit hoher Stabilität gegenüber starken Oxidationsmitteln. Auch unter diesen extremen Anwendungsbedingungen sollen diese Farbstoffe ihre positiven Echtheits- und Färbeeigenschaften nicht verlieren.

Aufgabe der vorliegenden Anmeldung ist es daher, Färbemittel für keratinische Fasern, insbesondere menschliche Haare, mit neuartigen direktziehenden Farbstoffen bereitzustellen, die hinsichtlich der Farbtiefe und der Echtheitseigenschaften, wie insbesondere Licht-, Reib- und Waschechtheit sowie Schweiß- und Kaltwellechtheit, gute anwendungstechnische Eigenschaften besitzen. Schließlich ist besonders wünschenswert, Färbemittel mit gutem Egalisiervermögen bereitzustellen. Im Falle der gleichzeitigen Anwendung mit Oxidationsfarbstoffen und/oder Oxidationsmitteln sollen die direktziehenden Farbstoffe eine ausreichende Stabilität gegen Wasserstoffperoxid und andere Oxidationsmittel besitzen und ihre positiven Echtheits- und Färbeeigenschaften nicht verlieren. Zudem sollten möglichst leuchtende und intensive Färbungen erzielt werden.

Es wurde überraschenderweise gefunden, dass sich bestimmte anionische Azofarbstoffe, die eine über eine Linkereinheit mit dem Azochromophor verbundene Sulfat-Einheit aufweisen, sehr gut als direktziehende Farbstoffe für die Haarfärbung eignen. In Ausfärbungen werden intensive Farbnuancen mit sehr guten Echtheitseigenschaften erhalten, insbesondere auch in Anwesenheit von Oxidationsmitteln.

Diese direktziehenden Farbstoffe liefern ebenfalls bei gleichzeitiger Anwendung von Oxidationsmitteln wie Wasserstoffperoxid oder einem Gemisch aus Wasserstoffperoxid und Peroxodisulfaten (Persulfate) intensive Nuancen ohne Abschwächung von Farbintensität und Farbbrillanz. Auf diese Weise wird das gleichzeitige Aufhellen und Färben von Haaren möglich, wodurch auch auf dunklem Haar eine leuchtende Farbgebung erzielt werden kann.

Aus EP 1915984 sind Azofarbstoffe, die eine kationische, spezifisch substituierte Thiazol-Einheit enthalten, als Haarfärbemittel zur Erzieleung dunkler, insbesondere schwarzer, Färbungen bekannt. Die darin genannten Farbstoffmoleküle enthalten eine Phenylazo-Einheit, die eine Alkylsubstitution mit Schwefelsäure-Funktion aufweisen kann. Zur Erzeugung von besonders leuchtenden Färbungen sind die darin genannten Farbstoffe nicht geeignet.

Färbemittel, enthaltend Verbindungen vom Typ der Azofarbstoffe gemäß nachstehender Formel (I), sind bislang nicht als Haarfarbstoffe bekannt.

Ein erster Gegenstand der vorliegenden Erfindung ist ein Mittel zum Färben und gegebenenfalls gleichzeitigen Aufhellen von Keratinfasern, insbesondere menschlichen Haaren, welches dadurch gekennzeichnet ist, es in einem kosmetischen Träger mindestens eine Verbindung der Formel (I) enthält, 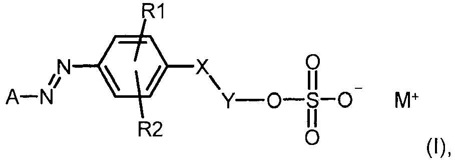 in welcher
- R1, R2: unabhängig voneinander für Wasserstoff, eine C₁-C₆-Alkylgruppe, eine C₂-C₆-Alkenylgruppe, eine Hydroxygruppe, ein Halogen, eine C₁-C₆-Alkoxygruppe, eine Aminogruppe, eine Nitrogruppe, eine Acetylaminogruppe oder eine Sulfonamidgruppe stehen, oder R1 und R2 bilden, sofern sie sich in ortho-Position zueinander befinden, einen 5- oder 6-gliedrigen, gesättigten oder ungesättigten Ring, der gegebenenfalls weitere Heteroatome enthalten kann,
- X: für O oder N-R3 stehen,
- R3: für Wasserstoff, eine C₁-C₆-Alkylgruppe, eine C₂-C₆-Alkenylgruppe, eine C₂-C₆-Hydroxy-alkylgruppe, eine C₂-C₆-Polyhydroxyalkylgruppe, eine Cyano-C₁-C₆-alkylgruppe oder eine Gruppe -Y'-O-SO₂-O- M⁺ steht,
- Y, Y': jeweils unabhängig voneinander für (CH₂)ₙ oder C₂H₄-(OC₂H₄)ₙ oder (CH₂)ₙ-O-(CH₂)ₘ oder (CH₂)ₙ-N(R6')-(CH₂)ₘ stehen und n und gegebenenfalls m jeweils eine ganze Zahl von 1 bis 6 stehen,
- A: für eine der Strukturen (II) bis (XII), (XIV) oder (XV) steht,

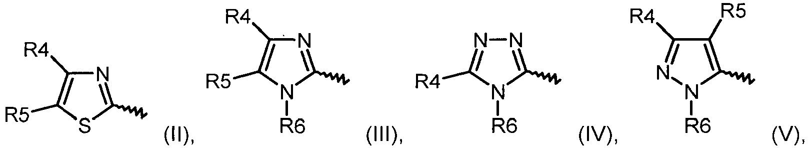

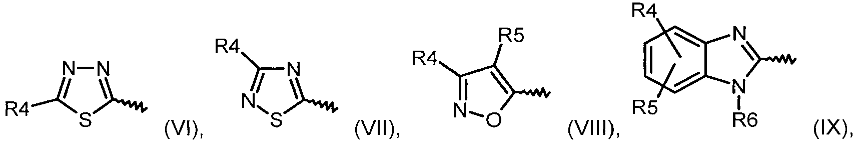

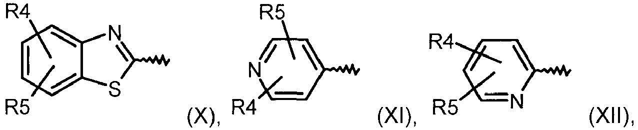

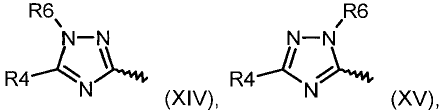

- R4, R5: jeweils unabhängig voneinander für Wasserstoff, eine Aminogruppe, eine C₁-C₆-Alkyl-aminogruppe, eine Di-(C₁-C₆-alkyl)aminogruppe, eine Hydroxygruppe, eine C₁-C₆-Alkoxygruppe, eine C₁-C₆-Alkylgruppe, eine C₂-C₆-Alkenylgruppe, eine Carbonsäuregruppe, eine Sulfonsäuregruppe, Halogen, eine Acetylaminogruppe oder eine Sulfonamidgruppe stehen, oder R4 und R5 bilden, sofern sie sich in ortho-Position zueinander befinden, einen 5- oder 6-gliedrigen, gesättigten oder ungesättigten Ring, der gegebenenfalls weitere Heteroatome enthalten kann,
- R6, R6': jeweils unabhängig voneinander für ein Wasserstoffatom, eine C₁-C₆-Alkylgruppe, eine C₂-C₆-Alkenylgruppe oder eine C₂-C₆-Hydroxyalkylgruppe stehen,
- und M⁺: für ein Proton (H⁺), ein Alkalimetallkation oder ein halbes Äquivalent eines Erdalkalimetallkations steht.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen von Keratinfasern geeignet sind, steht prinzipiell einer Verwendung auch auf anderen Gebieten nichts entgegen.

Der erfindungsgemäß verwendete Begriff "Färben von Keratinfasern" umfasst jedwede Form der Farbveränderung der Fasern. Umfasst sind insbesondere die unter den Begriffen Tönung, Blondierung, oxidativer Färbung, semipermanenter Färbung, permanenter Färbung sowie temporärer Färbung umfassten Farbveränderungen. Explizit mit umfasst sind erfindungsgemäß Farbveränderungen, die ein helleres Farbergebnis im Vergleich zur Ausgangsfarbe aufweisen, wie beispielsweise färbende Blondierungen.

Die erfindungsgemäßen Mittel enthalten die Verbindungen der Formel (I) in einem kosmetischen Träger. Dieser kosmetische Träger ist bevorzugt wässrig, alkoholisch oder wässrig-alkoholisch. Zum Zwecke der Haarbehandlung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch möglich, zur Lagerung eine pulverförmige oder auch tablettenförmige Formulierung bereitzustellen. Diese wird dann vor der Anwendung in einem wässrigen Lösungsmittel oder mit organischen Lösungsmitteln bzw. mit Gemischen aus Wasser und organischen Lösungsmitteln unter Erhalt der Anwendungsmischung vermengt. Ein wässriger Träger enthält im Sinne der Erfindung mindestens 40 Gew.-%, insbesondere mindestens 50 Gew.-% Wasser. Unter wässrigalkoholischen Trägern sind im Sinne der vorliegenden Erfindung wasserhaltige Zusammensetzungen, enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Bevorzugte erfindungsgemäße Mittel sind dadurch gekennzeichnet, dass sie zusätzlich ein nichtwässriges Lösungsmittel enthalten, wobei bevorzugte erfindungsgemäße Mittel das Lösungsmittel in einer Konzentration von 0,1 bis 30 Gew.-%, bevorzugt in einer Konzentration von 1 bis 20 Gew.-%, ganz besonders bevorzugt in einer Konzentration von 2 bis 10 Gew.-%, jeweils bezogen auf das Mittel, enthalten.

Im Folgenden werden Beispiele für die in Formel (I) genannten Substituenten R1, R2, R3, R4, R5, R6 und R6' exemplarisch genannt:
Beispiele für C₁-C₆-Alkylgruppen sind -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃, -(CH₂)₄CH₃, -(CH₂)₅CH₃. Besonders bevorzugte Alkylreste sind Methyl und Ethyl. Beispiele für C₂-C₆-Alkenylgruppen sind Vinyl, Prop-2-enyl (Allyl), 2-Methyl-prop-2-enyl, But-3-enyl, But-2-enyl, Pent-4-enyl oder Pent-3-enyl. Beispiele für C₂-C₆-Hydroxyalkylgruppen sind -CH₂CH₂OH, -CH₂CH₂CH₂OH, -CH₂CH(OH)CH₃, -CH₂CH₂CH₂CH₂OH, wobei die Gruppe -CH₂CH₂OH bevorzugt ist. Beispiele für C₂-C₆-Polyhydroxyalkylgruppen sind 1,2-Dihydroxyethyl, 2,3-Dihydroxypropyl, 3,4-Dihydroxybutyl und die 2,4-Dihydroxybutyl. Beispiele für C₁-C₆-Alkoxy-Gruppen sind Methoxy und Ethoxy, bevorzugt Methoxy. Beispiele für Cyano-C₁-C₆-alkylgruppen sind Cyanomethyl und 2-Cyanoethyl. Beispiele für Halogen sind Fluor, Chlor, Brom oder Iod, insbesondere Fluor und Chlor. Beispiele für C₁-C₆-Alkoxygruppen sind Methoxy oder Ethoxy. Beispiele für C₁-C₆-Alkylaminogruppen sind Methylamino, Ethylamino und Propylamino. Beispiele für Di(C₁-C₆-alkyl)-aminogruppen sind Dimethylamino, Diethylamino und Dipropylamino.

Bevorzugte Reste A sind Thiazol-Gruppen (II), Imidazol-Gruppen (III), Triazol-Gruppen (IV)/(XIV), Thiadiazol-gruppen (VI)/(VII) und Benzothiazol-Gruppen (X).

Eine weitere Ausführungsform des ersten Erfindungsgegenstands ist ein erfindungsgemäßes Mittel, welches dadurch gekennzeichnet ist, dass es eine Verbindung der Formel (I) enthält, in welcher A für eine der Strukturen (II), (III), (IV), (VI), (VII), (X), oder (XIV), insbesondere für (II), (IV) oder (X), steht.

In einer bevorzugten Ausführungsform steht A für eine 1,3-Thiazol-2-yl-Gruppe (II), bevorzugt für eine 1,3-Thiazol-2-yl-gruppe, worin R4 und R5 jeweils für Wasserstoff stehen.

In einer bevorzugten Ausführungsform steht A für eine Imidazol-2-yl-Gruppe (III), bevorzugt für eine Imidazol-2-yl-gruppe, worin R4, R5 und R6 jeweils für Wasserstoff stehen.

In einer bevorzugten Ausführungsform steht A für eine 4H-1,2,4-Triazol-3-yl-Gruppe (IV), bevorzugt für eine 4H-1,2,4-Triazol-3-yl-Gruppe, worin R4 und R6 jeweils für Wasserstoff stehen. In einer bevorzugten Ausführungsform steht A für eine 4H-1,2,4-Triazol-3-yl-Gruppe (IV), bevorzugt für eine 4H-1,2,4-Triazol-3-yl-Gruppe, worin R4 für Wasserstoff und R6 für Methyl stehen.

In einer bevorzugten Ausführungsform steht A für eine 1H-1,2,4-Triazol-3-yl-Gruppe (XIV), bevorzugt für eine 1 H-1,2,4-Triazol-3-yl-Gruppe, worin R4 und R6 jeweils für Wasserstoff stehen. In einer bevorzugten Ausführungsform steht A für eine 1H-1,2,4-Triazol-3-yl-Gruppe (XIV), bevorzugt für eine 1 H-1,2,4-Triazol-3-yl-Gruppe, worin R4 für Wasserstoff und R6 für Methyl stehen.

In einer bevorzugten Ausführungsform steht A für eine 1,3,4-Thiadiazol-2-yl-Gruppe (VI), bevorzugt für eine 1,3,4-Thiadiazol-2-yl-Gruppe, worin R4 für Wasserstoff steht.

In einer bevorzugten Ausführungsform steht A für eine 1,2,4-Thiadiazol-5-yl-Gruppe (VII), bevorzugt für eine 1,2,4-Thiadiazol-5-yl-Gruppe, worin R4 für Wasserstoff steht.

Weiterhin werden sehr gute Ergebnisse erhalten, wenn die Sulfateinheit über eine Aminogruppe an die Phenylazo-Einheit angebunden ist, bevorzugt eine Aminoalkyl-Einheit.

In einer weiteren Ausführungsform des ersten Erfindungsgegenstands enthält das Mittel eine Verbindung gemäß Formel (I), in welcher X für N-R3 und Y für (CH₂)ₙ mit n gleich 2 oder 3 stehen. Die erfindungsgemäßen Mittel können als Verbindung der Formel (I) für den Fall, dass X für N-R3 steht, auch zweifach ungeladene Verbindungen halten, sofern R3 für eine Gruppe -Y'-O-SO₂-O⁻ M⁺ steht. Erfindungsgemäß bevorzugte Verbindungen der Formel (I) enthalten jedoch nur eine anionische Gruppierung. Bevorzugt stehen X für N-R3 und R3 für eine C₁-C₆-Alkylgruppe.

Eine weitere Ausführungsform des ersten Erfindungsgegenstands ist ein Mittel, welches dadurch gekennzeichnet ist, dass es eine Verbindung der Formel (I) enthält, in welcher X für N-R3 und R3 für eine C₁-C₆-Alkylgruppe, insbesondere für Methyl oder Ethyl, stehen.

In einer bevorzugten Ausführungsform enthält das erfindungsgemäße Mittel mindestens eine Verbindung der Formel (I), in der A für eine 1,3-Thiazol-2-yl-gruppe (II), worin R4 und R5 jeweils für Wasserstoff stehen, X für N-R3, Y für (CH₂)ₙ mit n gleich 2, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine 1,3-Thiazol-2-yl-gruppe (II), worin R4 und R5 jeweils für Wasserstoff stehen, X für N-R3, Y für (CH₂)ₙ mit n gleich 3, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine Imidazol-2-yl-gruppe (III), worin R4, R5 und R6 jeweils für Wasserstoff stehen, X für N-R3, Y für (CH₂)ₙ mit n gleich 2, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine Imidazol-2-yl-gruppe (III), worin R4, R5 und R6 jeweils für Wasserstoff stehen, X für N-R3, Y für (CH₂)ₙ mit n gleich 3, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine 4H-1,2,4-Triazol-3-yl-Gruppe (IV), worin R4 und R6 jeweils für Wasserstoff stehen, X für N-R3, Y für (CH₂)ₙ mit n gleich 2, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für 4H-1,2,4-Triazol-3-yl-Gruppe (IV), worin R4 und R6 jeweils für Wasserstoff stehen, X für N-R3, Y für (CH₂)ₙ mit n gleich 3, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine 4H-1,2,4-Triazol-3-yl-Gruppe (IV), worin R4 für Wasserstoff und R6 für Methyl stehen, X für N-R3, Y für (CH₂)ₙ mit n gleich 2, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für 4H-1,2,4-Triazol-3-yl-Gruppe (IV), worin R4 für Wasserstoff und R6 für Methyl stehen, X für N-R3, Y für (CH₂)ₙ mit n gleich 3, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine 1H-1,2,4-Triazol-3-yl-Gruppe (XIV), worin R4 und R6 jeweils für Wasserstoff stehen, X für N-R3, Y für (CH₂)ₙ mit n gleich 2, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für 1H-1,2,4-Triazol-3-yl-Gruppe (XIV), worin R4 und R6 jeweils für Wasserstoff stehen, X für N-R3, Y für (CH₂)ₙ mit n gleich 3, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine 1H-1,2,4-Triazol-3-yl-Gruppe (XIV), worin R4 für Wasserstoff und R6 für Methyl stehen, X für N-R3, Y für (CH₂)ₙ mit n gleich 2, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für 1H-1,2,4-Triazol-3-yl-Gruppe (XIV), worin R4 für Wasserstoff und R6 für Methyl stehen, X für N-R3, Y für (CH₂)ₙ mit n gleich 3, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine 1,3,4-Thiadiazol-2-yl-Gruppe (VI), worin R4 für Wasserstoff steht, X für N-R3, Y für (CH₂)ₙ mit n gleich 2, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine 1,3,4-Thiadiazol-2-yl-Gruppe (VI), worin R4 für Wasserstoff steht, X für N-R3, Y für (CH₂)ₙ mit n gleich 3, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine 1,2,4-Thiadiazol-5-yl-Gruppe (VI), worin R4 für Wasserstoff steht, X für N-R3, Y für (CH₂)ₙ mit n gleich 2, und R3 für eine C₁-C₆-Alkylgruppe, bevorzugt Methyl oder Ethyl, stehen.

In einer bevorzugten Ausführungsform enthält das erfindungsgemäße Mittel mindestens eine Verbindung der Formel (I), in der A für eine 1,2,4-Thiadiazol-5-yl-Gruppe (VI), worin R4 für Wasserstoff steht, X für N-R3, Y für (CH₂)ₙ mit n gleich 3, und R3 für eine C₁-C₆-Alkylgruppe, bevorzugt Methyl oder Ethyl, stehen.

Weiterhin stehen die Reste R1 und R2 unabhängig voneinander bevorzugt für ein Wasserstoffatom, eine C₁-C₆-Alkylgruppe, ein Halogenatom oder eine C₁-C₆-Alkoxygruppe. Besonders bevorzugt ist es, wenn R1 und R2 unabhängig voneinander ein Wasserstoffatom, eine C₁-C₆-Alkylgruppe oder eine Nitrogruppe darstellen. Insbesondere stehen sowohl R1 als auch R2 für ein Wasserstoffatom oder einer der Reste R1 und R2 steht für Wasserstoff und der andere für eine Nitrogruppe.

Eine weitere Ausführungsform des ersten Erfindungsgegenstands ist daher ein Mittel, welches dadurch gekennzeichnet ist, dass es eine Verbindung der Formel (I) enthält, in welcher R1 und R2 unabhängig voneinander für Wasserstoff, eine C₁-C₆-Alkylgruppe oder eine Nitrogruppe, bevorzugt R1 und R2 jeweils für Wasserstoff oder bevorzugt R1 für Wasserstoff und R2 für eine Nitrogruppe, stehen.

In einer weiteren bevorzugten Ausführungsform stehen die Reste R4 und R5 unabhängig voneinander für ein Wasserstoffatom, eine C₁-C₆-Alkylgruppe, eine C₁-C₆-Alkoxygruppe, ein Halogenatom, eine C₁-C₆-Alkylsulfonylgruppe oder eine Nitrilgruppe.

In einer weiteren bevorzugten Ausführungsform stehen die Reste R6 und/oder R6' jeweils unabhängig voneinander für ein Wasserstoffatom oder eine C₁-C₆-Alkylgruppe, insbesondere für ein Wasserstoffatom oder eine Methylgruppe.

Erfindungsgemäß ist M eine Gruppe, die der Neutralisation der anionischen Schwefelsäureestergruppierung dient. Verbindungen, bei welchen M ein Proton (H⁺) oder ein Alkalimetallkation, insbesondere Natrium oder Kalium (Na⁺, K⁺) darstellt, erfüllen die erfindungsgemäße Aufgabenstellung in besonderem Maß und sind daher bevorzugt.

Erfindungsgemäß bevorzugte Mittel zum Färben und gegebenenfalls gleichzeitigen Aufhellen keratinischer Fasern sind dadurch gekennzeichnet, dass sie mindestens eine Verbindung der allgemeinen Formel (I) enthalten, ausgewählt aus der Gruppe
2-{Methyl[4-(1,3-thiazol-2-yldiazenyl)phenyl]amino}ethylhydrogensulfat, 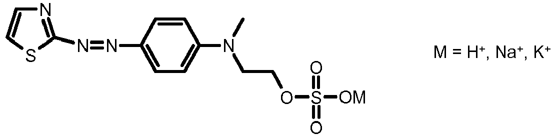
2-{Ethyl[4-(1,3-thiazol-2-yldiazenyl)phenyl]amino}ethylhydrogensulfat, 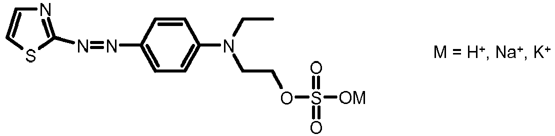
3-{Methyl[4-(1,3-thiazol-2-yldiazenyl)phenyl]amino}propylhydrogensulfat, 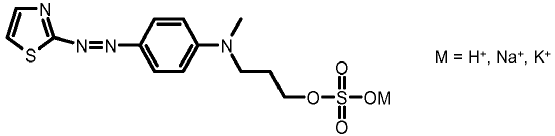
3-{Ethyl[4-(1,3-thiazol-2-yldiazenyl)phenyl]amino}propylhydrogensulfat 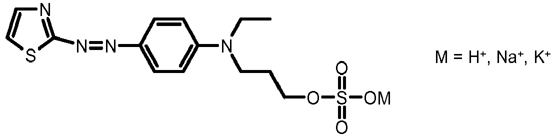
2-{Methyl[3-methyl-4-(1,3-thiazol-2-yldiazenyl)phenyl]amino}ethylhydrogensulfat, 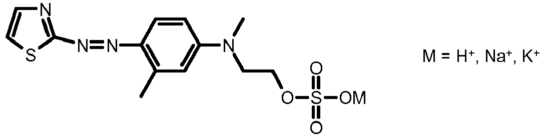
3-{Ethyl[3-methyl-4-(1,3-thiazol-2-yldiazenyl)phenyl]amino}propylhydrogensulfat, 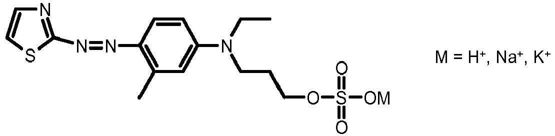
2-{[4-(1H-Imidazol-2-yldiazenyl)phenyl](methyl)amino}ethylhydrogensulfat, 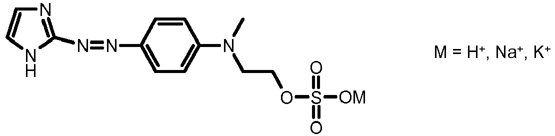
2-{Ethyl[4-(1H-imidazol-2-yldiazenyl)phenyl]amino}ethylhydrogensulfat, 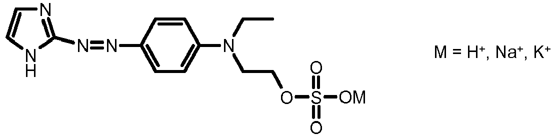
3-{Ethyl[4-(1H-imidazol-2-yldiazenyl)phenyl]amino}propylhydrogensulfat, 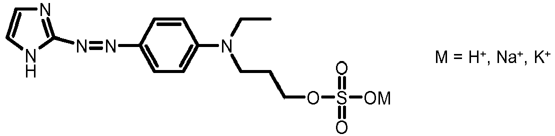
2-{Ethyl[4-(1H-imidazol-2-yldiazenyl)-3-methylphenyl]amino}ethylhydrogensulfat, 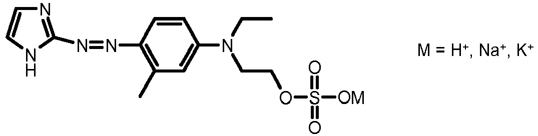
3-{Ethyl[4-(1H-imidazol-2-yldiazenyl)-3-methylphenyl]amino}propylhydrogensulfat, 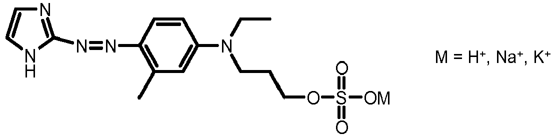
2-{Methyl[4-(4H-1,2,4-triazol-3-yldiazenyl)phenyl]amino}ethylhydrogensulfat, 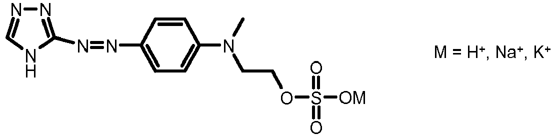
2-{Ethyl[4-(4H-1,2,4-triazol-3-yldiazenyl)phenyl]amino}ethylhydrogensulfat, 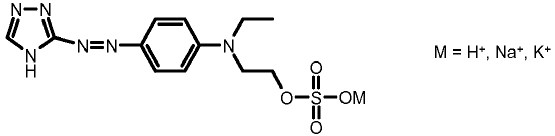
3-{Ethyl[4-(4H-1,2,4-triazol-3-yldiazenyl)phenyl]amino}propylhydrogensulfat, 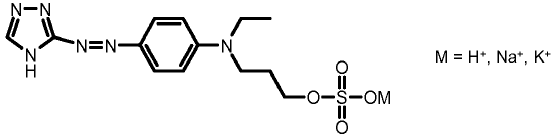
3-{Methyl[3-methyl-4-(4H-1,2,4-triazol-3-yldiazenyl)phenyl]amino}propylhydrogensulfat, 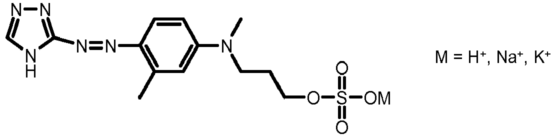
3-{Ethyl[3-methyl-4-(4H-1,2,4-triazol-3-yldiazenyl)phenyl]amino}propylhydrogensulfat, 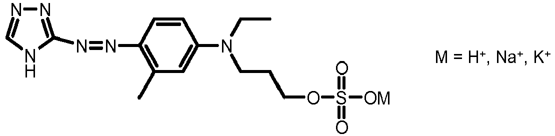
2-{Methyl[4-(1,3,4-thiadiazol-2-yldiazenyl)phenyl]amino}ethylhydrogensulfat, 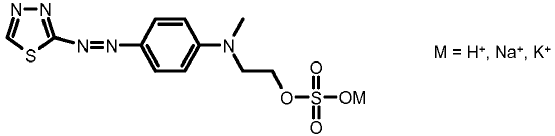
2-{Ethyl[4-(1,3,4-thiadiazol-2-yldiazenyl)phenyl]amino}ethylhydrogensulfat, 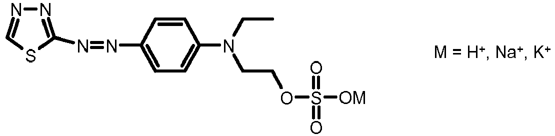
3-{Ethyl[4-(1,3,4-thiadiazol-2-yldiazenyl)phenyl]amino}propylhydrogensulfat, 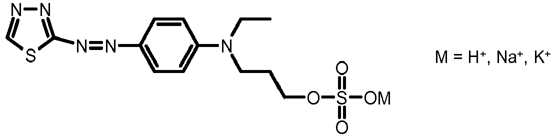
3-{Methyl[3-methyl-4-(1,3,4-thiadiazol-2-yldiazenyl)phenyl]amino}propylhydrogensulfat, 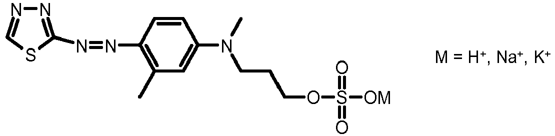
3-{Ethyl[3-methyl-4-(1,3,4-thiadiazol-2-yldiazenyl)phenyl]amino}propylhydrogensulfat, 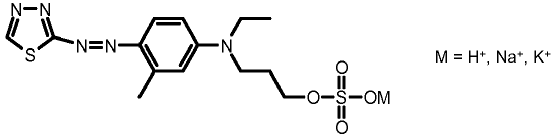
2-{Methyl[4-(1,2,4-thiadiazol-5-yldiazenyl)phenyl]amino}ethylhydrogensulfat, 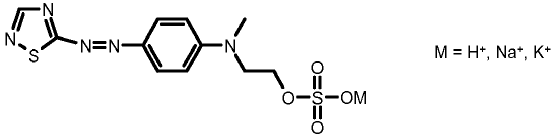
2-{Ethyl[4-(1,2,4-thiadiazol-5-yldiazenyl)phenyl]amino}ethylhydrogensulfat, 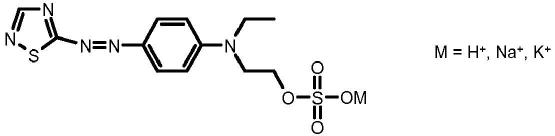
3-{Ethyl[4-(1,2,4-thiadiazol-5-yldiazenyl)phenyl]amino}propylhydrogensulfat, 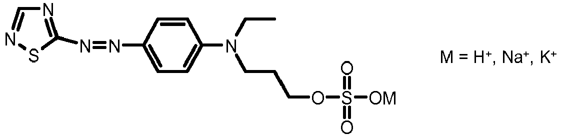
2-{Methyl[3-methyl-4-(1,2,4-thiadiazol-5-yldiazenyl)phenyl]amino}ethylhydrogensulfat, 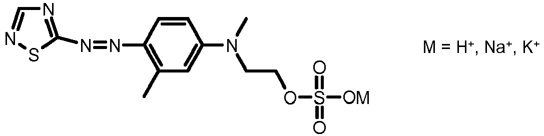
3-{Ethyl[3-methyl-4-(1,2,4-thiadiazol-5-yldiazenyl)phenyl]amino}propylhydrogensulfat, 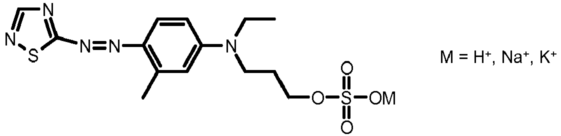
2-{Methyl[4-(1,3-benzothiazol-2-yldiazenyl)phenyl]amino}ethylhydrogensulfat, 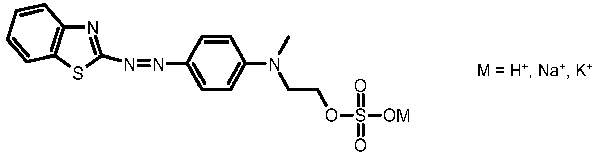
2-{Ethyl[4-(1,3-benzothiazol-2-yldiazenyl)phenyl]amino}ethylhydrogensulfat, 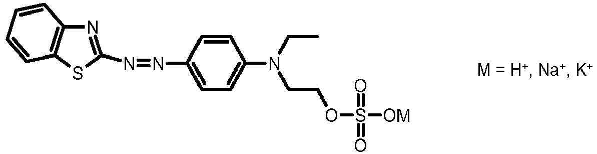
3-{Ethyl[4-(1,3-benzothiazol-2-yldiazenyl)phenyl]amino}propylhydrogensulfat, 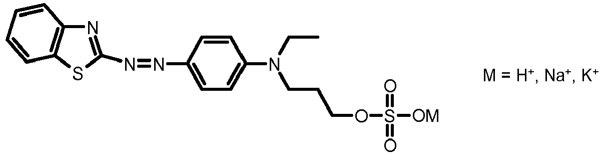
2-{[4-(1,3-Benzothiazol-2-yldiazenyl)-3-methylphenyl](methyl)amino}ethylhydrogensulfat, 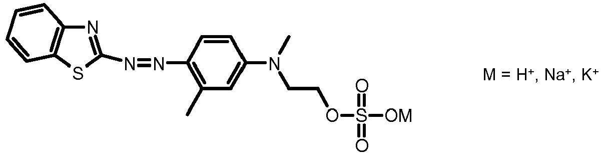
3-{Ethyl[4-(1,3-Benzothiazol-2-yldiazenyl)-3-methylphenyl]amino}propylhydrogensulfat, 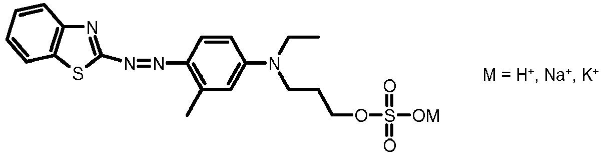
2-{Methyl[4-(1,3-benzimidazol-2-yldiazenyl)phenyl]amino}ethylhydrogensulfat, 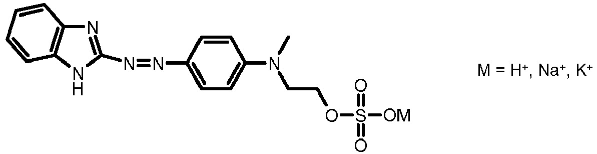
2-{[4-(1,3-Benzimidazol-2-yldiazenyl)phenyl](ethyl)amino}ethylhydrogensulfat, 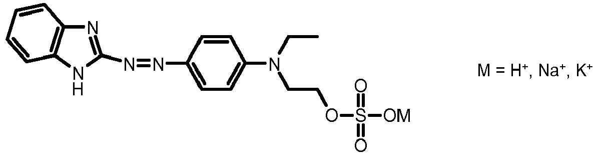
3-{Ethyl[4-(1,3-benzimidazol-2-yldiazenyl)phenyl]amino}propylhydrogensulfat, 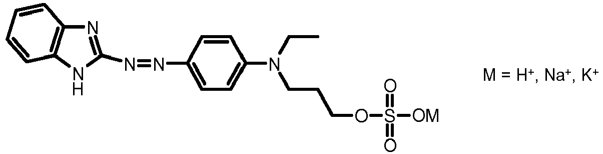
2-{[4-(1,3-Benzimidazol-2-yldiazenyl)-3-methylphenyl](methyl)amino}ethylhydrogensulfat, 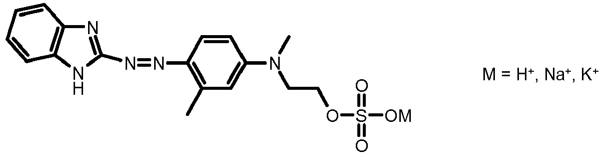
3-{[4-(1,3-Benzimidazol-2-yldiazenyl)-3-methylphenyl](ethyl)amino}propylhydrogensulfat, 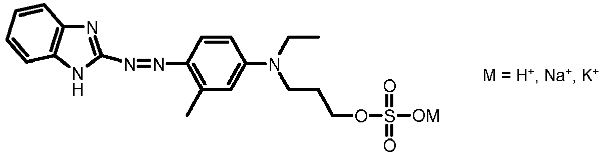
2-[Methyl({4-[(E)-2-(1,2-oxazol-5-yl)diazen-1-yl]phenyl})amino]ethylhydrogensulfat, 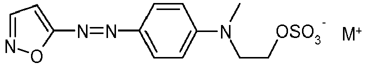
2-[Ethyl({4-[(E)-2-(1,2-oxazol-5-yl)diazen-1-yl]phenyl})amino]ethylhydrogensulfat, 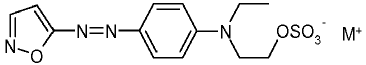
2-[Methyl({2-nitro-4-[2-(1,2-oxazol-5-yl)diazen-1-yl]phenyl})amino]ethylhydrogensulfat, 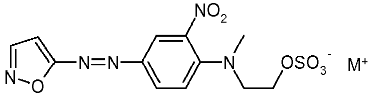
2-[Ethyl({2-nitro-4-[2-(1,2-oxazol-5-yl)diazen-1-yl]phenyl})amino]ethylhydrogensulfat, 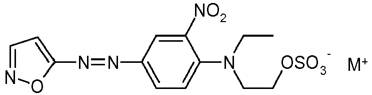
2-[Methyl({3-nitro-4-[2-(1,2-oxazol-5-yl)diazen-1-yl]phenyl})amino]ethylhydrogensulfat, 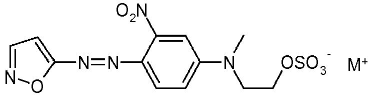
2-[Ethyl({3-nitro-4-[2-(1,2-oxazol-5-yl)diazen-1-yl]phenyl})amino]ethylhydrogensulfat, 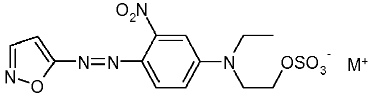
2-[Methyl({2-nitro-4-[2-(4H-1,2,4-triazol-3-yl)diazen-1-yl]phenyl})amino]ethylhydrogensulfat, 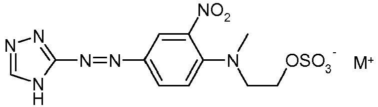
2-[Ethyl({2-nitro-4-[2-(4H-1,2,4-triazol-3-yl)diazen-1-yl]phenyl})amino]ethylhydrogensulfat, 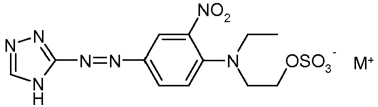
2-[Methyl({3-nitro-4-[2-(4H-1,2,4-triazol-3-yl)diazen-1-yl]phenyl})amino]ethylhydrogensulfat, 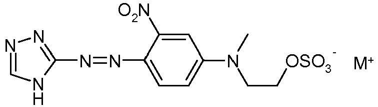
2-[Ethyl({3-[2-(4H-1,2,4-triazol-3-yl)diazen-1-yl]phenyl})amino]ethylhydrogensulfat, 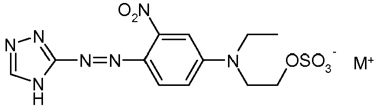
2-[Methyl({4-[2-(4-methyl-4H-1,2,4-triazol-3-yl)diazen-1-yl]-phenyl})amino]ethylhydrogensulfat, 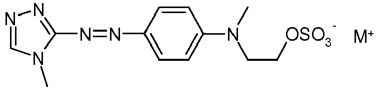
2-[Ethyl({4-[2-(4-methyl-4H-1,2,4-triazol-3-yl)diazen-1-yl]-phenyl})amino]ethylhydrogensulfat, 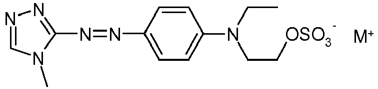
2-[Methyl({4-[2-(4-methyl-4H-1,2,4-triazol-3-yl)diazen-1-yl]-2-nitrophenyl})amino]ethylhydrogensulfat, 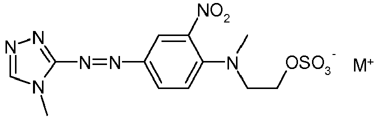
2-[Ethyl({4-[2-(4-methyl-4H-1,2,4-triazol-3-yl)diazen-1-yl]-2-nitrophenyl})amino]ethylhydrogensulfat, 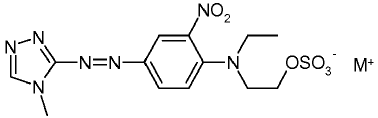
2-[Methyl({4-[2-(4-methyl-4H-1,2,4-triazol-3-yl)diazen-1-yl]-3-nitrophenyl})amino]ethylhydrogensulfat, 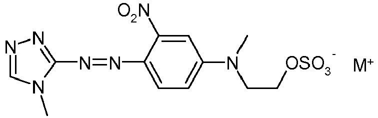
2-[Ethyl({4-[2-(4-methyl-4H-1,2,4-triazol-3-yl)diazen-1-yl]-3-nitrophenyl})amino]ethylhydrogensulfat, 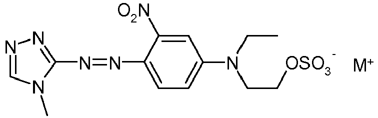
2-[Methyl({4-[2-(1-methyl-1H-1,2,4-triazol-3-yl)diazen-1-yl]phenyl})amino]ethylhydrogensulfat, 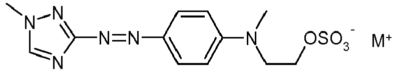
2-[Ethyl({4-[2-(1-methyl-1H-1,2,4-triazol-3-yl)diazen-1-yl]phenyl})amino]ethylhydrogensulfat, 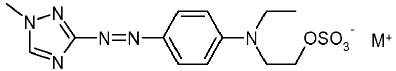
2-[Methyl({4-[2-(1-methyl-1H-1,2,4-triazol-3-yl)diazen-1-yl]-2-nitrophenyl})amino]ethylhydrogensulfat, 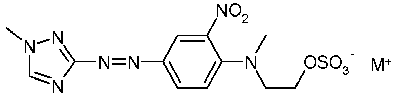
2-[Ethyl({4-[2-(1-methyl-1H-1,2,4-triazol-3-yl)diazen-1-yl]-2-nitrophenyl})amino]ethylhydrogensulfat, 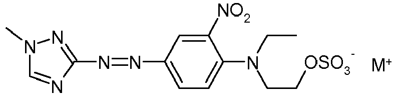
2-[Methyl({4-[2-(1-methyl-1H-1,2,4-triazol-3-yl)diazen-1-yl]-3-nitrophenyl})amino]ethylhydrogensulfat, 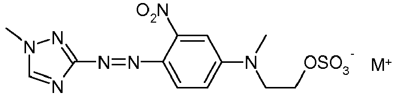
2-[Ethyl({4-[2-(1-methyl-1H-1,2,4-triazol-3-yl)diazen-1-yl]-3-nitrophenyl})amino]ethylhydrogensulfat, 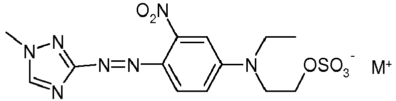
2-[Methyl({4-[2-(1,2,4-thiadiazol-5-yl)diazen-1-yl]phenyl})amino]ethylhydrogensulfat, 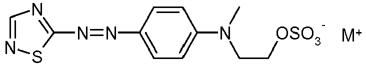
2-[Ethyl({4-[2-(1,2,4-thiadiazol-5-yl)diazen-1-yl]phenyl})amino]ethylhydrogensulfat, 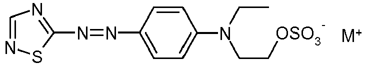
2-[Methyl({2-nitro-4-[2-(1,2,4-thiadiazol-5-yl)diazen-1-yl]phenyl})amino]ethylhydrogensulfat, 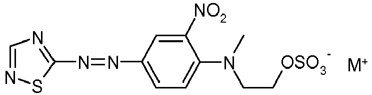
2-[Ethyl({2-nitro-4-[2-(1,2,4-thiadiazol-5-yl)diazen-1-yl]phenyl})amino]ethylhydrogensulfat, 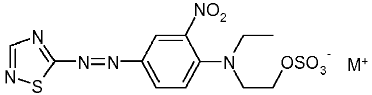
2-[Methyl({3-nitro-4-[2-(1,2,4-thiadiazol-5-yl)diazen-1-yl]phenyl})amino]ethylhydrogensulfat, 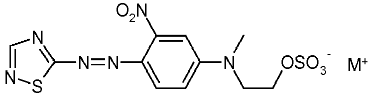
2-[Ethyl({3-nitro-4-[2-(1,2,4-thiadiazol-5-yl)diazen-1-yl]phenyl})amino]ethylhydrogensulfat, 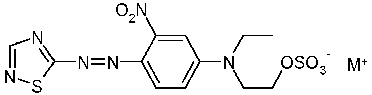
2-[Methyl({4-[2-(1,3,4-thiadiazol-2-yl)diazen-1-yl]phenyl})amino]ethylhydrogensulfat, 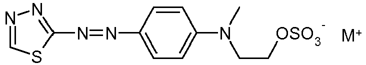
2-[Ethyl({4-[2-(1,3,4-thiadiazol-2-yl)diazen-1-yl]phenyl})amino]ethylhydrogensulfat, 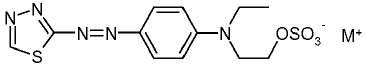
2-[Methyl({2-nitro-4-[2-(1,3,4-thiadiazol-2-yl)diazen-1-yl]phenyl})amino]ethylhydrogensulfat, 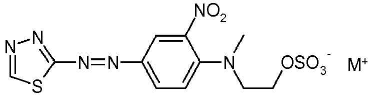
2-[Ethyl({2-nitro-4-[2-(1,3,4-thiadiazol-2-yl)diazen-1-yl]phenyl})amino]ethylhydrogensulfat, 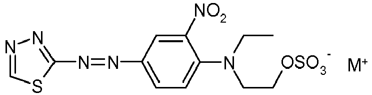
2-[Methyl({3-nitro-4-[2-(1,3,4-thiadiazol-2-yl)diazen-1-yl]phenyl})amino]ethylhydrogensulfat, 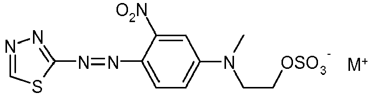
2-[Ethyl({3-nitro-4-[2-(1,3,4-thiadiazol-2-yl)diazen-1-yl]phenyl})amino]ethylhydrogensulfat, 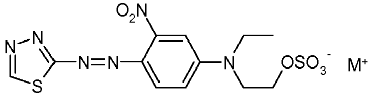
und/oder dem Natrium- oder Kaliumsalz einer der voranstehenden Verbindungen.

Eine besonders bevorzugte Ausführungsform des ersten Erfindungsgegenstands sind Mittel, welche als Verbindung der Formel (I) mindestens eine Verbindung, ausgewählt aus 2-{Methyl[4-(1,3-thiazol-2-yldiazenyl)phenyl]amino}ethylhydrogensulfat, 2-{Ethyl[4-(1,3-thiazol-2-yldiazenyl)-phenyl]amino}ethylhydrogensulfat, 3-{Methyl[4-(1,3-thiazol-2-yldiazenyl)phenyl]amino}propylhydrogensulfat, 3-{Ethyl[4-(1,3-thiazol-2-yldiazenyl)phenyl]amino}propylhydrogensulfat, 2-{Methyl[4-(4H-1,2,4-triazol-3-yldiazenyl)phenyl]amino}ethylhydrogensulfat, 2-{Ethyl[4-(4H-1,2,4-triazol-3-yldiazenyl)phenyl]amino}ethylhydrogensulfat, 3-{Ethyl[4-(4H-1,2,4-triazol-3-yldiazenyl)-phenyl]amino}propylhydrogensulfat, 2-{Methyl[4-(1,2,4-thiadiazol-5-yldiazenyl)phenyl]amino}-ethylhydrogensulfat, 2-{Ethyl[4-(1,2,4-thiadiazol-5-yldiazenyl)phenyl]amino}ethylhydrogensulfat, 3-{Ethyl[4-(1,2,4-thiadiazol-5-yldiazenyl)phenyl]amino}propylhydrogensulfat, 2-{Methyl[4-(1,3-benzothiazol-2-yldiazenyl)phenyl]amino}ethylhydrogensulfat, 2-{Ethyl[4-(1,3-benzothiazol-2-yldiazenyl)-phenyl]amino}ethylhydrogensulfat, 3-{Ethyl[4-(1,3-benzothiazol-2-yldiazenyl)phenyl]amino}-propylhydrogensulfat, 2-[Methyl({4-[(E)-2-(1,2-oxazol-5-yl)diazen-1-yl]phenyl})amino]ethylhydrogensulfat, 2-[Ethyl({4-[(E)-2-(1,2-oxazol-5-yl)diazen-1-yl]phenyl})amino]ethylhydrogensulfat, 2-[Ethyl({2-nitro-4-[2-(1,2-oxazol-5-yl)diazen-1-yl]phenyl})amino]ethylhydrogensulfat, 2-[Ethyl({3-nitro-4-[2-(1,2-oxazol-5-yl)diazen-1-yl]phenyl})amino]ethylhydrogensulfat, 2-[Ethyl({2-nitro-4-[2-(4H-1,2,4-triazol-3-yl)diazen-1-yl]phenyl})amino]ethylhydrogensulfat, 2-[Ethyl({3-nitro-4-[2-(4H-1,2,4-triazol-3-yl)diazen-1-yl]phenyl})amino]ethylhydrogensulfat, 2-[Methyl({4-[2-(4-methyl-4H-1,2,4-triazol-3-yl)diazen-1-yl]-phenyl})amino]ethylhydrogensulfat, 2-[Ethyl({4-[2-(4-methyl-4H-1,2,4-triazol-3-yl)diazen-1-yl]-phenyl})amino]ethylhydrogensulfat, 2-[Ethyl({4-[2-(4-methyl-4H-1,2,4-triazol-3-yl)diazen-1-yl]-2-nitrophenyl})amino]ethylhydrogensulfat, 2-[Ethyl({4-[2-(4-methyl-4H-1,2,4-triazol-3-yl)diazen-1-yl]-3-nitrophenyl})amino]ethylhydrogensulfat, 2-[Ethyl({2-nitro-4-[2-(1,2,4-thiadiazol-5-yl)diazen-1-yl]phenyl})amino]ethylhydrogensulfat, 2-[Ethyl({4-[2-(1-methyl-1H-1,2,4-triazol-3-yl)diazen-1-yl]phenyl})amino]ethylhydrogensulfat, 2-[Ethyl({4-[2-(1-methyl-1H-1,2,4-triazol-3-yl)diazen-1-yl]-2-nitrophenyl})amino]ethylhydrogensulfat, 2-[Ethyl({3-nitro-4-[2-(1,2,4-thiadiazol-5-yl)diazen-1-yl]phenyl})amino]ethylhydrogensulfat, 2-[Ethyl({2-nitro-4-[2-(1,3,4-thiadiazol-2-yl)diazen-1-yl]phenyl})amino]ethylhydrogensulfat, 2-[Ethyl({3-nitro-4-[2-(1,3,4-thiadiazol-2-yl)diazen-1-yl]phenyl})amino]ethylhydrogensulfat und/oder dem Natrium- oder Kaliumsalz einer der voranstehenden Verbindungen, enthalten.

Die erfindungsgemäßen Mittel zum Färben und gegebenenfalls gleichzeitigen Aufhellen von Keratinfasern enthalten die Verbindung(en) der Formel (I) vorzugsweise in Mengen oberhalb von 1 ppm und unterhalb von 10 Gew.-%, jeweils bezogen auf das gesamte Mittel. Eine bevorzugte Ausführungsform des ersten Erfindungsgegenstands ist dabei ein Mittel, welches dadurch gekennzeichnet ist, dass es die Verbindung(en) der Formel (I) in einem Anteil von 0,001 bis 5 Gew.-%, vorzugsweise von 0,0025 bis 2,5 Gew.-%, besonders bevorzugt von 0,005 bis 2,0 Gew.-% und insbesondere von 0,01 bis 1,5 Gew.-%, jeweils bezogen auf das Gesamtgewicht des Mittels, enthält.

Bevorzugte erfindungsgemäße Mittel zum Färben und gegebenenfalls gleichzeitigen Aufhellen von Keratinfasern enthalten mindestens eine Verbindung der Formel (I) in einer Gesamtmenge von 0,001 bis 5 Gew.-%, vorzugsweise von 0,0025 bis 2,5 Gew.-%, besonders bevorzugt von 0,005 bis 2,0 Gew.-% und insbesondere von 0,01 bis 1,5 Gew.-%, jeweils bezogen auf das Gesamtgewicht des Mittels.

In einer weiteren bevorzugten Ausführungsform enthalten die erfindungsgemäßen Mittel neben der Verbindung der Formel (I) zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen oder den Indophenolen und deren physiologisch verträglichen Salzen. Die zusätzlichen direktziehenden Farbstoffe werden jeweils bevorzugt in einem Anteil von 0,001 bis 2 Gew.-%, bezogen auf das Gesamtgewicht des Mittels, eingesetzt.

Bevorzugte anionische direktziehende Farbstoffe sind die unter den internationalen Bezeichnungen bzw. Handelsnamen Bromphenolblau, Tetrabromphenolblau, Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1 und Acid Black 52 bekannten Verbindungen.

Bevorzugte kationische direktziehende Farbstoffe sind Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), Basic Blue 99, Basic Brown 16 und Basic Brown 17 sowie Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Die Färbemittel können weiterhin als aufhellende Färbemittel eingesetzt werden. Zur Erzielung des Aufhelleffekts enthalten die Mittel dazu Wasserstoffperoxid und/oder ein festes Anlagerungsprodukt von Wasserstoffperoxid an organische oder anorganische Verbindungen. Beispiele für solche Anlagerungsprodukte sind Anlagerungsprodukte von Wasserstoffperoxid an Harnstoff, Melamin sowie Natriumborat.

Zur Erzielung einer verstärkten Aufhell- und Bleichwirkung kann das Mittel weiterhin mindestens ein Peroxo-Salz enthalten. Geeignete Peroxo-Salze sind anorganische Peroxoverbindungen, bevorzugt ausgewählt aus der Gruppe, gebildet aus Ammoniumperoxodisulfat, Alkalimetallperoxodisulfaten, Ammoniumperoxomonosulfat, Alkalimetallperoxomonosulfaten, Alkalimetallperoxodiphosphaten und Erdalkalimetallperoxiden. Besonders bevorzuge sind Peroxodisulfate, insbesondere Ammoniumperoxodisulfat, Kaliumperoxodisulfat und Natriumperoxodisulfat.

Eine weitere Ausführungsform des ersten Erfindungsgegenstands ist daher ein Mittel zum Färben und gleichzeitigen Aufhellen keratinischer Fasern, das dadurch gekennzeichnet ist, dass es zusätzlich mindestens ein Oxidationsmittel, ausgewählt aus Natriumpersulfat, Kaliumpersulfat, Ammoniumpersulfat, Wasserstoffperoxid und/oder einem der festen Anlagerungsprodukte von Wasserstoffperoxid an organische oder anorganische Verbindungen und Gemischen der voranstehenden Verbindungen, enthält.

In einer bevorzugten Ausführungsform wird Wasserstoffperoxid selbst als wässrige Lösung verwendet. Die Konzentration einer Wasserstoffperoxid-Lösung im erfindungsgemäßen Mittel wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte anwendungsbereite Mittel des ersten Erfindungsgegenstands sind dadurch gekennzeichnet, dass sie 0,5 bis 15 Gew.-%, vorzugsweise 1 bis 12,5 Gew.-%, besonders bevorzugt 2,5 bis 10 Gew.-% und insbesondere 3 bis 6 Gew.-% Wasserstoffperoxid, jeweils bezogen auf das Gesamtgewicht des anwendungsbereiten Mittels, enthalten.

Unter anwendungsbereitem Mittel versteht man das Färbemittel so, wie es auf die keratinischen Fasern aufgetragen wird. Bei Mitteln zum Färben und gleichzeitigen Aufhellen keratinischer Fasern umfasst das erfindungsgemäße anwendungsbereite Mittel also mindestens eine Verbindung der Formel (I) und mindestens ein Oxidationsmittel, ausgewählt aus Natriumpersulfat, Kaliumpersulfat, Ammoniumpersulfat, Wasserstoffperoxid und/oder einem der festen Anlagerungsprodukte von Wasserstoffperoxid an organische oder anorganische Verbindungen sowie aus Gemischen der voranstehenden Verbindungen.

Wenn die Mittel zusätzlich Persulfate enthalten, so sind diese Persulfate in dem Mittel bevorzugt bezogen zu 1,5 bis 60 Gew.-%, vorzugsweise 2,0 bis 45 Gew.-%, besonders bevorzugt 2,5 bis 40 Gew.-% und insbesondere zu 5 bis 30 Gew.-% , jeweils bezogen auf das Gesamtgewicht des Mittels, enthalten.

Das Mittel kann zur Verstärkung der Blondierwirkung weitere Bleichkraftverstärker enthalten, wie z. B. Tetraacetylethylendiamin (TAED), 1,5-Diacetyl-2,4-dioxohexahydro-1,3,5-triazin (DADHT), Tetraacetylglykoluril (TAGU), N-Nonanoylsuccinimid (NOSI), n-Nonanoyl- oder Isononanoyloxybenzolsulfonat (n- bzw. i-NOBS), Phthalsäureanhydrid, Triacetin, Ethylenglykoldiacetat und 2,5-Diacetoxy-2,5-dihydrofuran sowie Carbonatsalze bzw. Hydrogencarbonatsalze, insbesondere Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat, und stickstoffhaltige, heterocyclische Bleichkraftverstärker, wie 4-Acetyl-1-methylpyridinium-p-toluolsulfonat, 2-Acetyl-1-methylpyridinium-p-toluolsulfonat sowie N-Methyl-3,4-dihydroisochinolinium-p-toluolsulfonat.

Zur weiteren Steigerung der Aufhellung kann der erfindungsgemäßen Zusammensetzung zusätzlich mindestens eine SiO₂-Verbindung, wie Kieselsäure oder Silicate, insbesondere Wassergläser, zugesetzt sein. Es kann erfindungsgemäß bevorzugt sein, die SiO₂-Verbindungen in Mengen von 0,05 Gew.-% bis 15 Gew.-%, besonders bevorzugt in Mengen von 0,15 Gew.-% bis 10 Gew.-% und ganz besonders bevorzugt in Mengen von 0,2 Gew.-% bis 5 Gew.-%, jeweils bezogen auf die erfindungsgemäße Zusammensetzung, einzusetzen. Die Mengenangaben geben dabei jeweils den Gehalt der SiO₂-Verbindungen ohne deren Wasseranteil in den Mitteln wieder. Die erfindungsgemäßen Mittel können weiterhin auch als Oxidationsfärbemittel eingesetzt werden. Solche Oxidationsfärbemittel enthalten zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ. Besonders geeignete Oxidationsfarbstoffvorprodukte vom Entwickler-Typ sind dabei ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)-phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind dabei ausgewählt aus der Gruppe, gebildet aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis (2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl) amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

Die Entwicklerkomponenten und Kupplerkomponenten werden bevorzugt in einem Anteil von 0,0001 bis 5,0 Gew.-%, vorzugsweise 0,001 bis 2,5 Gew.-%, jeweils bezogen auf das anwendungsbereite Mittel, verwendet. Dabei werden Entwicklerkomponenten und Kupplerkomponenten im Allgemeinen in etwa äquimolaren Mengen zueinander eingesetzt. Wenn sich auch der äquimolare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 zu 0,5 bis 1 zu 3, insbesondere 1 zu 1 bis 1 zu 2, stehen können.

Im Falle von Oxidationsfärbemitteln enthalten die Mittel bevorzugt ein Oxidationsmittel, bevorzugt Wasserstoffperoxid. Die Mengen an Wasserstoffperoxid entsprechen den Mengen in den erfindungsgemäßen Aufhellmitteln.

Die anwendungsbereiten Färbemittel können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die anwendungsbereiten Färbemittel als flüssige Zubereitung bereitgestellt und den Mitteln daher zusätzlich eine oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, kationischen, zwitterionischen, amphoteren und nichtionischen Tensiden und Emulgatoren ausgewählt.

Erfindungsgemäß bevorzugte Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß bevorzugte Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß bevorzugte Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden bevorzugt in Anteilen von 0,1 bis 45 Gew.%, besonders bevorzugt 1 bis 30 Gew.% und ganz besonders bevorzugt von 1 bis 15 Gew.%, bezogen auf die Gesamtmenge der anwendungsbereiten Mittel, eingesetzt.

Erfindungsgemäß geeignete Mittel können auch kationische Tenside vom Typ der quartären Ammoniumverbindungen, der Esterquats und der Amidoamine enthalten. Bevorzugte quaternäre Ammoniumverbindungen sind Ammoniumhalogenide sowie die unter den INCI-Bezeichnungen Quaternium-27 und Quaternium-83 bekannten Imidazolium-Verbindungen. Weitere erfindungsgemäß verwendbare kationische Tenside stellen die quaternisierten Proteinhydrolysate dar. Eine erfindungsgemäß besonders geeignete Verbindung aus der Amidoamine stellt das unter der Bezeichnung Tegoamid^{®} S 18 im Handel erhältliche Stearamidopropyl-dimethylamin dar. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Die kationischen Tenside sind in den erfindungsgemäß verwendeten Mitteln bevorzugt in Anteilen von 0,05 bis 10 Gew.-%, bezogen auf das gesamte Mittel, enthalten.

Die anwendungsbereiten Färbemittel können weitere Hilfs- und Zusatzstoffe enthalten. So hat es sich als vorteilhaft erwiesen, wenn die Mittel mindestens ein Verdickungsmittel enthält. Bezüglich dieser Verdickungsmittel bestehen keine prinzipiellen Einschränkungen. Es können sowohl organische als auch rein anorganische Verdickungsmittel zum Einsatz kommen.

Geeignete Verdickungsmittel sind anionische, synthetische Polymere; kationische, synthetische Polymere; natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen; nichtionische, synthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels zwischen 6 und 11, insbesondere zwischen 7 und 10,5, liegt. Bei den pH-Werten im Sinne der vorliegenden Erfindung handelt es sich um pH-Werte, die bei einer Temperatur von 22 °C gemessen wurden.

Die zur Einstellung des bevorzugten pH-Wertes erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)-Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Magnesiumcarbonat, Natriumhydroxid, Kaliumhydroxid, Natriumsilicat und Natriummetasilicat. Erfindungsgemäß einsetzbare, organische Alkalisierungsmittel werden bevorzugt ausgewählt aus Monoethanolamin, 2-Amino-2-methylpropanol und Triethanolamin. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin.

Weiterhin hat es sich als vorteilhaft erweisen, wenn die Färbemittel, insbesondere wenn sie zusätzlich Wasserstoffperoxid enthalten, mindestens einen Stabilisator oder Komplexbildner enthalten. Besonders bevorzugte Stabilisatoren sind Phenacetin, Alkalibenzoate (Natriumbenzoat) und Salicylsäure. Weiterhin können alle Komplexbildner des Standes der Technik eingesetzt werden. Erfindungsgemäß bevorzugte Komplexbildner sind stickstoffhaltigen Polycarbonsäuren, insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Ferner können die erfindungsgemäßen Mittel weitere Wirk-, Hilfs- und Zusatzstoffe enthalten, wie nichtionische Polymere (beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane); Silikone, wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere); kationische Polymere wie quaternisierte Celluloseether, Polysiloxane mit quaternären Gruppen wie Dimethyldiallylammoniumchlorid-Copolymere, mit Diethylsulfat quaternierte Dimethylamino-ethylmethacrylat-Vinylpyrrolidinon-Copolymere, Vinylimidazoli- i-nium-methochlorid-Copolymere; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Zur Anwendung der erfindungsgemäßen Mittel eignet sich insbesondere ein Verfahren zum Färben von keratinischen Fasern, insbesondere menschlichen Haaren, welches dadurch gekennzeichnet ist, dass ein Mittel des ersten Erfindungsgegenstands auf die keratinhaltigen Fasern aufgebracht, 5 bis 60 Minuten auf der Faser belassen und anschließend mit Wasser wieder ausgespült oder mit einem Shampoo ausgewaschen wird. Bevorzugt beträgt die Einwirkzeit der anwendungsbereiten Färbemittel 5 bis 45 min, insbesondere 10 bis 40 min, besonders bevorzugt 15 bis 35 min. Während der Einwirkzeit des Mittels auf der Faser kann es vorteilhaft sein, den Färbevorgang und/oder Aufhellvorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung und/oder Haaraufhellung am lebenden Probanden, durch die Körpertemperatur des Probanden erfolgen. Bei letzterer Möglichkeit wird üblicherweise die zu färbende und/ oder aufzuhellende Partie mit einer Haube abgedeckt. Eine Einwirkphase bei Raumtemperatur ist ebenfalls erfindungsgemäß. Insbesondere liegt die Temperatur während der Einwirkzeit zwischen 20 °C und 40 °C, insbesondere zwischen 25 °C und 38 °C. Nach Ende der Einwirkzeit wird die verbleibende Färbezubereitung mit Wasser oder einem Reinigungsmittel aus dem Haar gespült. Als Reinigungsmittel kann dabei insbesondere handelsübliches Shampoo dienen, wobei insbesondere dann auf das Reinigungsmittel verzichtet werden kann und der Ausspülvorgang mit Wasser erfolgen kann, wenn das Färbemittel und/oder Aufhellmittel einen stark tensidhaltigen Träger besitzt.

Die erfindungsgemäßen Mittel können als Einkomponentenmittel (Färbe- und Aufhellmittel) oder als Mehrkomponentenmittel wie Zweikomponenten-Mittel oder Dreikomponenten-Mittel formuliert und entsprechend angewendet werden. Eine Auftrennung in Mehrkomponentensysteme bietet sich insbesondere dort an, wo Inkompatibilitäten der Inhaltsstoffe zu erwarten oder zu befürchten sind; das einzusetzende Mittel wird bei solchen Systemen vom Verbraucher direkt vor der Anwendung durch Vermischen der Komponente hergestellt.

Ein Färbe- und Aufhellverfahren, bei dem die Aufhellcreme und das Oxidationsmittel zunächst getrennt vorliegen, ist dabei bevorzugt. Ein weiterer Gegenstand der vorliegenden Erfindung ist daher ein Verfahren zum Färben und Aufhellen von menschlichen Haaren, bei dem eine Zusammensetzung auf wässriger Grundlage, enthaltend Wasserstoffperoxid, mit einem erfindungsgemäßen Mittel des ersten Erfindungsgegenstands zu einer homogenen Zusammensetzung (vorstehend auch als "anwendungsbereites Mittel" bezeichnet) vermischt und diese auf das Haar aufgebracht wird.

Bezüglich weiterer bevorzugter Ausführungsformen der erfindungsgemäßen Verfahren gilt mutatis mutandis das zu den erfindungsgemäßen Mitteln Gesagte.

Die Verbindungen der allgemeinen Formel (I) eignen sich sehr gut als direktziehende Farbstoffe für die Haarfärbung mit hoher Farbintensität und Farbbrillanz. In Ausfärbungen werden intensive und natürliche Farbnuancen mit sehr guten Echtheitseigenschaften. Es ist auf diese Weise auch das gleichzeitige Aufhellen und Färben von Haaren möglich, wodurch auch auf dunklem Haar eine leuchtende Farbgebung erzielt werden kann.

Die direktziehenden Farbstoffe liefern in Färbemitteln bei gleichzeitiger Anwendung von Oxidationsmitteln wie Wasserstoffperoxid oder einem Gemisch aus Wasserstoffperoxid und Peroxodisulfaten intensive Nuancen ohne Abschwächung von Farbintensität und Farbbrillanz. Auf diese Weise wird das gleichzeitige Aufhellen und Färben von Haaren möglich, wodurch auch auf dunklem Haar eine Farbgebung erzielt werden kann.

Ein weiterer Gegenstand der vorliegenden Erfindung ist die kosmetische Verwendung eines Mittels des ersten Erfindungsgegenstands in Mitteln zur Färbung menschlicher Haare
zur Verbesserung der Farbintensität und/oder
zur Verbesserung der Echtheitseigenschaften der Färbung, insbesondere der Waschechtheit, der Lichtechtheit und der Ultrablondierechtheit.

Bezüglich weiterer bevorzugter Ausführungsformen der erfindungsgemäßen Verwendung gilt mutatis mutandis das zu den erfindungsgemäßen Mitteln Gesagte.

Schließlich sind die Verbindungen gemäß Formel (I) bislang im Stand der Technik unbekannt. Ein weiterer Gegenstand der vorliegenden Erfindung ist daher eine Verbindung der Formel (I), entsprechend den Ausführungen des ersten Erfindungsgegenstands.

### Beispiele - Synthesebeispiele

### Synthesebeispiel 1: Synthese von 2-{Ethyl[4-(1,3-thiazol-2-yldiazenyl)phenyl]amino}ethylsulfat, Natriumsalz (DZ 1)

### 1.1. Synthese von 2-[Ethyl(phenyl)amino]ethylhydrogensulfat

In 25,0 ml konzentrierte Schwefelsäure wurden bei einer Temperatur von 35 - 40 °C 16,5 g (0,10 mol) 2-(N-Ethylanilino)ethanol gegeben. Die Reaktion verlief leicht exotherm und wurde durch Kühlung mit Eiswasser im angegebenen Temperaturbereich gehalten. Das Gemisch wurde für weitere 2 h gerührt. Nach der Beendigung der Reaktion wurde auf Eis gegossen und durch Zugabe von Wasser auf 250 ml aufgefüllt. Es resultierte eine durchsichtig gelbe Lösung, die ohne weitere Aufarbeitung in der nachfolgenden Stufe eingesetzt wurde.

### 1.2. Synthese von 2-{Ethyl[4-(1,3-thiazol-2-yldiazenyl)phenyl]amino}ethylsulfat, Natriumsalz (DZ 1)

10,0 g (0,10 mol) 2-Aminothiazol wurden in 300 ml 60 Gew.-%ige, wässrige Essigsäurelösung gegeben. Nach dem Hinzufügen von 20 ml konzentrierter Schwefelsäure wurde das resultierende Gemisch auf 5 °C gekühlt. Anschließend wurden 40 ml Nitrosylschwefelsäure (40 Gew.-%ig) tropfenweise hinzugefügt, unter Kühlung auf 0 °C wurde für 2 h nachgerührt.

Zu der zuvor frisch hergestellten Diazoniumsalz-Lösung wurde anschließend unter Kühlung auf 10 °C das unter 1.1 hergestellte Rohprodukt getropft. Bei Raumtemperatur wurde der Ansatz über Nacht nachgerührt. Dann wurde durch Zugabe einer 50 %igen wässrigen Natronlauge-Lösung ein pH-Wert von 7 eingestellt. Hierbei fiel ein roter Feststoff aus, welcher abfiltriert und getrocknet wurde. Zur Abtrennung von den noch vorhandenen anorganischen Salzen wurde der Feststoff unter Erwärmen mit Ethanol ausgerührt und filtriert. Anschließend wurde das Filtrat wieder komplett eingeengt. Ausbeute: 31,4 g (88,2 %); ¹H-NMR (400 MHz, DMSO-d₆): δ [ppm] = 1,17 (s, 3H); 3,56 (q, 2H); 3,70 (t, 2H); 3,94 (t, 2H); 6,92 (d, 2H); 7,68 (d, 1H); 7,79 (d, 2H); 7,92 (d, 1H); ¹³C-NMR (400 MHz, DMSO-d6): δ [ppm] = 12,4; 45,7; 49,8; 63,5; 112,3; 120,5; 126,9; 141,7; 143,5; 152,5; 178,2.

### Synthesebeispiel 2: Synthese von 2-{Ethyl[4-(4H-1,2,4-triazol-3-yldiazenyl)phenyl]amino}ethylsulfat, Natriumsalz (DZ 2)

### 2.1. Synthese von 2-[Ethyl(phenyl)aminolethylhydrogensulfat

Vgl. 1.1

### 2.2. Synthese von 2-{Ethyl[4-(4H-1,2,4-triazol-3-yldiazenyl)phenyl]amino}ethylsulfat, Natriumsalz (DZ 2)

8,4 g (0,10 mol) 3-Amino-1H-1,2,4-triazol wurden in 300 ml Gew.-60 %ige Essigsäurelösung gegeben. Nach dem Hinzufügen von 20 ml konzentrierter Schwefelsäure wurde das resultierende Gemisch auf 5 °C gekühlt. Anschließend wurden 40 ml Nitrosylschwefelsäure (40 Gew.-%ig) tropfenweise hinzugefügt und für 2 h unter Kühlung auf 0 °C gerührt.

Zu der zuvor frisch hergestellten Diazoniumsalz-Lösung wurde anschließend unter Kühlung auf 10 °C das unter 2.1 hergestellte Rohprodukt der Kupplungskomponente getropft. Bei Raumtemperatur wurde der Ansatz über Nacht nachgerührt. Dann wurde durch Zugabe einer 50 %igen wässrigen Natronlauge-Lösung ein pH-Wert von 7 eingestellt. Hierbei fiel ein oranger Feststoff aus, welcher abfiltriert und getrocknet wurde. Zur Abtrennung von den noch vorhandenen anorganischen Salzen wurde der Feststoff unter Erwärmen mit Ethanol ausgerührt und filtriert. Anschließend wurde das Filtrat wieder komplett eingeengt. Ausbeute: 8,8 g (26,0 %); ¹H-NMR (400 MHz, DMSO-d₆): δ [ppm] = 1,15 (s, 3H); 3,44 (q, 2H); 3,69 (t, 2H); 4,20 (t, 2H); 6,71 (d, 2H); 7,60 (d, 2H); 8,29 (s, 1H); ¹³C-NMR (400 MHz, DMSO-d6): δ [ppm] =14,7; 48,6; 51,9; 69,0; 114,5; 129,6; 144,8; 148,6; 155,4; 184,0.

### Synthesebeispiel 3: Synthese von 2-{Ethyl[4-(1,3-benzothiazol-2-yldiazenyl)phenyl]amino}ethylsulfat, Natriumsalz (DZ 3)

### 3.1. Synthese von 2-[Ethyl(phenyl)aminolethylhydropensulfat

Vgl. 1.1

### 3.2. Synthese von 2-{Ethyl[4-(4-(1,3-benzothiazol-2-yldiazenyl)phenyl]amino}ethylsulfat, Natriumsalz (DZ 3)

15,0 g (0,10 mol) 2-Aminobenzothiazol wurden in 300 ml 60 Gew.-%ige Essigsäurelösung gegeben. Nach dem Hinzufügen von 20 ml konzentrierter Schwefelsäure wurde das resultierende Gemisch auf 5 °C gekühlt. Anschließend wurden 40 ml Nitrosylschwefelsäure (40 Gew.-%ig) tropfenweise hinzugefügt und für 2 h unter Kühlung auf 0 °C gerührt.

Zu der zuvor frisch hergestellten Diazoniumsalz-Lösung wurde anschließend unter Kühlung auf 10 °C das unter 2.1 hergestellte Rohprodukt der Kupplungskomponente getropft. Bei Raumtemperatur wurde der Ansatz über Nacht nachgerührt. Dann wurde durch Zugabe einer 50 %igen wässrigen Natronlauge-Lösung ein pH-Wert von 7eingestellt. Hierbei fiel ein oranger Feststoff aus, welcher abfiltriert und getrocknet wurde. Zur Abtrennung von den noch vorhandenen anorganischen Salzen wurde der Feststoff unter Erwärmen mit Ethanol ausgerührt und filtriert. Anschließend wurde das Filtrat wieder komplett eingeengt. Ausbeute: 19,8 g (48,9 %).

### Ausfärbungsbeispiele:

### 1.1 Herstellung der Färbecremes

Es wurde folgende Färbecremes hergestellt:

**1.1.1 Nichtionische Färbecreme 1**

| | |
|---|---|
| Cetearyl Alcohol | 6,0 g |
| Coconut Alcohol | 6,0 g |
| PEG-40 Hydrogenated Castor Oil | 1,0 g |
| Ceteareth-12 | 3,0 g |
| Ceteareth-20 | 3,0 g |
| PHB-Methylester | 0,3 9 |
| PHB-Propylester | 0,2 g |
| Phenoxyethanol | 1,0 g |
| PEG-8 | 5,0 g |
| Erfindungsgemäßer DZ | 1,0 g |
| Ammoniumsulfat | 1,0 g (in 30,0 g Wasser) |
| Hydroxyethylcellulose | 1,0 g (in 15,0 g Wasser) |
| NaOH 0,1% | Ad pH-Wert |
| Wasser | ad 100 g |

Die ersten neun Komponenten wurden zusammen bei 80 °C aufgeschmolzen, danach wurde der in wenig Wasser vorgelöste Farbstoff zugefügt. Diese Mischung wurde mit einer Lösung des Ammoniumsulfats in 30 g Wasser emulgiert. Anschließend wurde eine Quellung von 1,0 g Hydroxyethylcellulose in 15,0 g Wasser hinzugegeben. Der in der Tabelle 2 angegebene pH-Wert wurde mit 0,1% Natronlauge eingestellt, anschließend wurde mit Wasser auf 100 g aufgefüllt.

**1.1.2 Kationische Färbecreme 2**

| | |
|---|---|
| Cetearyl Alcohol | 4,0 g |
| Ceteareth-12 | 1,0 g |
| Dehyquart^{®} A-CA | 2,0 g |
| Ammoniumsulfat | 1,0 g (in 30,0 g Wasser) |
| Erfindungsgemäßer DZ | 1,0 g |
| Wasser | ad 100 g |

Cetearyl Alcohol wurde zusammen mit Ceteareth-12 und Dehyquart^{®} A-CA aufgeschmolzen, danach wurde die Schmelze mit heißem Wasser emulgiert. Dann wurden der in wenig Wasser vorgelöste Farbstoff sowie die wässrige Ammoniumsulfatlösung hinzugegeben. Der pH-Wert wurde mit Ammoniak bzw. Zitronensäure auf den in der Tabelle angegebenen Wert eingestellt, anschließend wurde mit Wasser wurde auf 100 g aufgefüllt.

**1.1.3 Anionische Färbecreme 3**

| | |
|---|---|
| Cetearyl Alcohol | 1,0 g |
| Coconut Alcohol | 1,0 g |
| Akypo Soft^{®} RLM 45N | 1,1 g |
| PHB-Propylester | 0,1 g |
| PHB-Methylester | 0,1 g |
| Ammoniumsulfat | 1,0 g (in 30,0 g Wasser) |
| Erfindungsgemäßer DZ | 1,0 g |
| Wasser | ad 100 g |

Die ersten fünf Komponenten wurden zusammen aufgeschmolzen. Diese Schmelze wurde mit heißem Wasser emulgiert, anschließend wurde der in Wasser vorgelöste Farbstoff hinzugegeben sowie die Ammoniumsulfatlösung hinzugefügt. Der in der Tabelle angegebene pH-Wert wurde mit Ammoniak bzw. Zitronensäure eingestellt, danach wurde mit Wasser auf 100 g aufgefüllt.

### 1.2 Verzeichnis der eingesetzten Rohstoffe

- Akypo RLM 45 NV^{®}: Laurylalkohol-4.5-EO-Essigsäure-Natrium-Salz (min. 22% Aktiv-substanzgehalt; INCI-Bezeichnung: Sodium Laureth-5 Carboxylate)
- Dehyquart^{®} A-CA: Trimethylhexadecylammoniumchlorid (ca. 24 -26 % Aktivsubstanz; INCI-Bezeichnung: Aqua (Water), Cetrimonium Chloride)

### 1.3 Ausfärbungen

Jeweils 1,8 g der Färbecreme wurden auf eine ca. 6 cm lange Strähne Menschenhaares (Kerling Euronaturhaar, blond) aufgetragen und dort 30 min bei 30°C belassen. Nach Beendigung der Einwirkzeit wurde das Haar ausgespült, mit einem üblichen Haarwaschmittel gewaschen und anschließend getrocknet. Die Haarsträhnen wurden in den in der nachfolgenden Tabelle angegebenen Nuancen gefärbt.
DZ 1: 2-{Ethyl[4-(1,3-thiazol-2-yldiazenyl)phenyl]amino}ethylsulfat, Natriumsalz
DZ 2: 2-{Ethyl[4-(4H-1,2,4-triazol-3-yldiazenyl)phenyl]amino}ethylsulfat, Natriumsalz
DZ 3: 2-{Ethyl[4-(1,3-benzothiazol-2-yldiazenyl)phenyl]amino}ethylsulfat, Natriumsalz

| Farbstoff | Färbecreme | pH-Wert | Farbnuance | Intensität |
|---|---|---|---|---|
| DZ 1 | 1 | 7,0 | krebsrot | +++ |
| DZ 1 | 1 | 9,5 | granatrot | +++ |
| DZ 1 | 2 | 7,0 | granatrot | +++ |
| DZ 1 | 2 | 9,5 | lackrot | +++ |
| DZ 1 | 3 | 7,0 | korallenrot | +++ |
| DZ 1 | 3 | 9,5 | lackrot | +++ |
| DZ 2 | 1 | 7,0 | orange | +++ |
| DZ 2 | 1 | 9,5 | rotorange | +++ |
| DZ 2 | 2 | 7,0 | aprikosengelb | +++ |
| DZ 2 | 2 | 9,5 | orange | +++ |
| DZ 2 | 3 | 7,0 | gelborange | +++ |
| DZ 2 | 3 | 9,5 | orange | +++ |
| DZ 3 | 1 | 7,0 | mattrot | ++ |
| DZ 3 | 1 | 9,5 | granatrot | ++ |
| DZ 3 | 2 | 7,0 | graurot | ++ |
| DZ 3 | 2 | 9,5 | rot | ++ |
| DZ 3 | 3 | 7,0 | graurot | ++ |
| DZ 3 | 3 | 9,5 | portweinrot | ++ |

| | | | | |
|---|---|---|---|---|
| Intensität: + = niedrig ++ = mittel +++ = hoch | | | | |

### 1.4 Waschechtheit

Mit der unter 1.1.3 erhaltenen Färbecreme 3 mit pH 9,5, enthaltend DZ1, wurden Haarsträhnen ausgefärbt (Kerling, Euronaturhaar weiß), getrocknet und farbmetrisch vermessen (Spectralflasch SF 450, Datacolor). Zur Bestimmung der Waschechheit wurden die Strähnen 6 mal per Hand gewaschen. Hierzu wurde die Strähne zunächst mit Wasser angefeuchtet und anschließend jeweils für 45 s mit einer 25 %igen Texapon NSO-UP Lösung shampooniert. Das shampoonierte Haar wurde 1 min lang mit handwarmem Wasser gründlich ausgespült. Anschließend wurde die Haarsträhne mit einem Fön gertrocknet. Nach dem Trocknen erfolgte der nächste Waschvorgang. Nach 6 Haarwäschen wurden die Strähnen erneut farbmetrisch vermessen.

DZ 1: 2-{Ethyl[4-(1,3-thiazol-2-yldiazenyl)phenyl]amino}ethylsulfat, Natriumsalz

| DZ1 | Kerling (Euronaturhaar weiß) | | |
|---|---|---|---|
| | L | a | b |
| 0 Haarwäschen | 39,15 | 43,59 | 25,98 |
| 6 Haarwäschen | 44,95 | 44,78 | 25,32 |

Sowohl beim visuellen Vergleich der ungewaschenen gegen die gewaschene Haarsträhne als auch beim Vergleich der farbmetrischen Daten lässt sich eine ausgezeichnete Waschechtheit der erfindungsgemäßen Verbindung feststellen.

### 1.5 Überprüfung der Lichtechtheit (nach DIN 54004)

Die mit Färbecreme 3 nach dem unter 1.3 beschriebenen Verfahren gefärbten Haarsträhnen wurden für 120 Stunden in einem Xenotestgerät (Hanau) belichtet. Gleichzeitig wurden als Referenz textile Lichtechtheitsmaßstäbe (mit Lichtechtheitswerten von 1 bis 6) mit belichtet. Nach Beendigung der Belichtung erfolgte die Bewertung der Lichtechtheit (LE) der erfindungsgemäßen Verbindung durch visuellen Abgleich mit den textilen Lichtechtheitsmaßstäben. Ab einem Lichtechtheitswert von 3 wird die Lichtechtheit eines direktziehenden Farbstoffes als gut beurteilt.

DZ 1: 2-{Ethyl[4-(1,3-thiazol-2-yldiazenyl)phenyl]amino}ethylsulfat, Natriumsalz

| | |
|---|---|
| Färbecreme 3, DZ1 | LE = 4 |

| | |
|---|---|
| LE = 1 (sehr schlechte Lichtechtheit) LE = 6 (sehr gute Lichtechtheit) | |

### 1.6 Überprüfung der Ultrablondierechtheit

Es wurde die folgende Blondiercreme hergestellt:

| Beschreibung | Gew.-% |
|---|---|
| Cetearyl Alcohol | 11,00 |
| Coconut Alcohol | 2,75 |
| Ceteareth-20 | 0,25 |
| Sodium Laureth Sulfate, 27 Gew.-% | 26,50 |
| Ammoniumsulfat | 1,00 |
| Etidronic Acid, 60 Gew.-% | 0,20 |
| Sodium Silicate | 0,50 |
| Weizenproteinhydrolysat, 40 Gew.-% | 0,35 |
| DZ 1: 2-{Ethyl[4-(1,3-thiazol-2-yldiazenyl)phenyl]amino}ethylsulfat, Natriumsalz | 2,00 |
| Vitamin F | 1,00 |
| Ammoniak, 25 Gew.-% | 7,60 |
| Parfum | qs |
| Wasser | Ad 100 |

Die Blondiercreme wurde im Verhältnis 1:1 mit folgender Oxidationsmitteldispersion vermischt:

| Beschreibung | Gew.-% |
|---|---|
| Sodium Hydroxide, 45 Gew.-% | 0,73 |
| Dipicolinsäure | 0,10 |
| Dinatriumpyrophosphat | 0,03 |
| Etidronic Acid, 60 Gew.-% | 1,50 |
| Sodium Laureth Sulfate, 27 Gew.-% | 2,00 |
| Dimethicone, 10 Gew.-% | 0,07 |
| Acrylates Copolymer ca. 30 Gew.-% | 12,00 |
| Wasserstoffperoxid, 50 Gew.-% | 22,40 |
| Wasser | Ad 100 |

Anschließend wurde dieses Gemisch mit 0,37 Anteilen Ammoniumpersulfat vermengt (Ammoniumpersulfat-Gehalt von 27,0 Gew.-% in der Anwendungsmischung). Nach dem Homogenisieren des Gemisches wurde ein anwendungsbereites Booster-Blondiermittel erhalten, wovon jeweils 1,8 g auf eine ca. 6 cm lange Strähne Menschenhaares (Kerling Euronaturhaar, blond) aufgetragen und dort 30 min bei 30 °C belassen wurden. Nach Beendigung der Einwirkzeit wurde das Haar ausgespült, mit einem üblichen Haarwaschmittel gewaschen und anschließend getrocknet. Es wurde die folgende Färbung erhalten:

DZ 1: 2-{Ethyl[4-(1,3-thiazol-2-yldiazenyl)phenyl]amino}ethylsulfat, Natriumsalz

| Farbstoff | Färbecreme | pH-Wert | Farbnuance | Intensität |
|---|---|---|---|---|
| DZ 1 | 3 | 9,5 | lackrot | +++ |

| | | | | |
|---|---|---|---|---|
| Intensität: + = niedrig ++ = mittel +++ = hoch | | | | |

Das auf diese Weise erhaltene Färbeergebnis wurde visuell mit den unter 1.3. erhaltenen Färbeergebnissen verglichen. Der Vergleich zeigte, dass bei Ausfärbung in Gegenwart eines Gemisches aus Wasserstoffperoxid und Persulfaten eine brillante Färbung erhalten werden konnte, die gegenüber der Formulierung ohne Oxidationsmittel keine Abschwächung in der Farbintensität aufwies.

## Patentansprüche

1. Mittel zum Färben von keratinischen Fasern, insbesondere menschlichen Haaren, **dadurch gekennzeichnet, dass** es in einem kosmetischen Träger mindestens eine Verbindung der Formel (I) enthält, 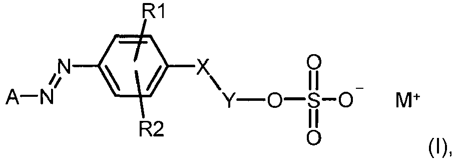 in welcher
R1, R2 unabhängig voneinander für Wasserstoff, eine C₁-C₆-Alkylgruppe, eine C₂-C₆-Alkenylgruppe, eine Hydroxygruppe, ein Halogen, eine C₁-C₆-Alkoxygruppe, eine Aminogruppe, eine Nitrogruppe, eine Acetylaminogruppe oder eine Sulfonamidgruppe stehen, oder R1 und R2 bilden, sofern sie sich in ortho-Position zueinander befinden, einen 5- oder 6-gliedrigen, gesättigten oder ungesättigten Ring, der gegebenenfalls weitere Heteroatome enthalten kann,
X für O oder N-R3 stehen,
R3 für Wasserstoff, eine C₁-C₆-Alkylgruppe, eine C₂-C₆-Alkenylgruppe, eine C₂-C₆-Hydroxyalkylgruppe, eine C₂-C₆-Polyhydroxyalkylgruppe, eine Cyano-C₁-C₆-alkyl-gruppe oder eine Gruppe -Y'-O-SO₂-O- M⁺ steht,
Y, Y' jeweils unabhängig voneinander für (CH₂)ₙ oder C₂H₄-(OC₂H₄)ₙ oder (CH₂)ₙ-O-(CH₂)ₘ oder (CH₂)ₙ-N(R6')-(CH₂)ₘ stehen und n und gegebenenfalls m jeweils eine ganze Zahl von 1 bis 6 stehen,
A für eine der Strukturen (II) bis (XII), (XIV) oder (XV) steht, 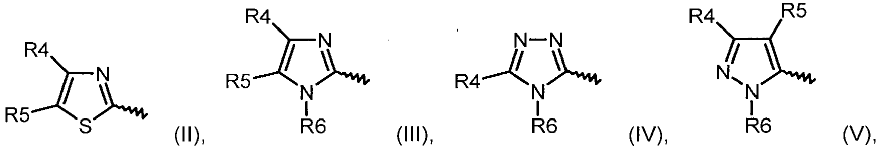 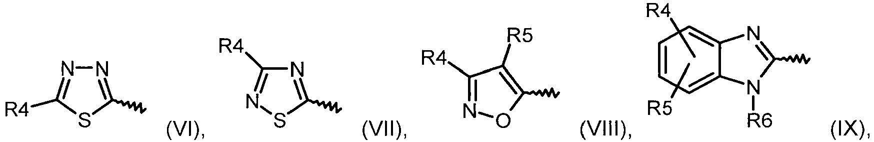 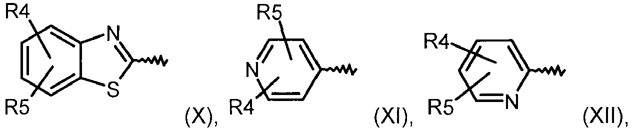 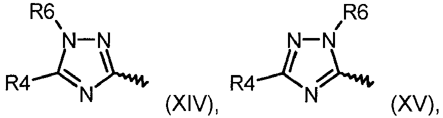
R4, R5 jeweils unabhängig voneinander für Wasserstoff, eine Aminogruppe, eine C₁-C₆-Alkylaminogruppe, eine Di-(C₁-C₆-alkyl)aminogruppe, eine Hydroxygruppe, eine C₁-C₆-Alkoxygruppe, eine C₁-C₆-Alkylgruppe, eine C₂-C₆-Alkenylgruppe, eine Carbonsäuregruppe, eine Sulfonsäuregruppe, Halogen, eine Acetylaminogruppe oder eine Sulfonamidgruppe stehen, oder R4 und R5 bilden, sofern sie sich in ortho-Position zueinander befinden, einen 5- oder 6-gliedrigen, gesättigten oder ungesättigten Ring, der gegebenenfalls weitere Heteroatome enthalten kann,
R6, R6' jeweils unabhängig voneinander für ein Wasserstoffatom, eine C₁-C₆-Alkylgruppe, eine C₂-C₆-Alkenylgruppe oder eine C₂-C₆-Hydroxyalkylgruppe stehen,
und M⁺ für ein Proton (H⁺), ein Alkalimetallkation oder ein halbes Äquivalent eines Erdalkalimetallkations steht.

2. Mittel nach Anspruch 1, **dadurch gekennzeichnet, dass** es mindestens eine Verbindung der Formel (I) enthält, in welcher A für eine der Strukturen (II), (III), (IV), (VI), (VII), (X) oder (XIV), insbesondere (II), (IV) oder (X) steht.

3. Mittel nach einem der Ansprüche 1 oder 2, **dadurch gekennzeichnet, dass** es mindestens eine Verbindung der Formel (I) enthält, in welcher X für N-R3 und Y für (CH₂)ₙ mit n gleich 2 oder 3 stehen.

4. Mittel nach einem der Ansprüche 1 bis 3, **dadurch gekennzeichnet, dass** es eine Verbindung der Formel (I) enthält, in welcher X für N-R3 und R3 für eine C₁-C₆-Alkylgruppe, insbesondere für Methyl oder Ethyl, stehen.

5. Mittel nach einem der Ansprüche 1 bis 4, **dadurch gekennzeichnet, dass** es eine Verbindung der Formel (I) enthält, in welcher R1 und R2 unabhängig voneinander für Wasserstoff, eine C₁-C₆-Alkylgruppe oder eine Nitrogruppe stehen.

6. Mittel nach einem der Ansprüche 1 bis 5, **dadurch gekennzeichnet, dass** es eine Verbindung der Formel (I) enthält, in welcher R1 und R2 jeweils für Wasserstoff stehen.

7. Mittel nach einem der Ansprüche 1 bis 5, **dadurch gekennzeichnet, dass** es eine Verbindung der Formel (I) enthält, in welcher R1 für Wasserstoff und R2 für eine Nitrogruppe, stehen.

8. Mittel nach einem der Ansprüche 1 bis 7, **dadurch gekennzeichnet, dass** es eine Verbindung der Formel (I) enthält, ausgewählt aus 2-{Methyl[4-(1,3-thiazol-2-yldiazenyl)phenyl]amino}ethylhydrogensulfat, 2-{Ethyl[4-(1,3-thiazol-2-yldiazenyl)phenyl]amino}ethylhydrogensulfat, 3-{Methyl[4-(1,3-thiazol-2-yldiazenyl)phenyl]amino}propylhydrogensulfat, 3-{Ethyl[4-(1,3-thiazol-2-yldiazenyl)phenyl]amino}propylhydrogensulfat, 2-{Methyl[3-methyl-4-(1,3-thiazol-2-yldiazenyl)phenyl]amino}ethylhydrogensulfat, 3-{Ethyl[3-methyl-4-(1,3-thiazol-2-yldiazenyl)phenyl]amino}propylhydrogensulfat, 2-{[4-(1H-Imidazol-2-yldiazenyl)phenyl](methyl)amino}ethylhydrogensulfat, 2-{Ethyl[4-(1H-imidazol-2-yldiazenyl)phenyl]amino}ethylhydrogensulfat, 3-{Ethyl[4-(1H-imidazol-2-yldiazenyl)phenyl]amino}propylhydrogensulfat, 2-{Ethyl[4-(1H-imidazol-2-yldiazenyl)-3-methylphenyl]amino}ethylhydrogensulfat, 3-{Ethyl[4-(1H-imidazol-2-yldiazenyl)-3-methylphenyl]amino}propylhydrogensulfat, 2-{Methyl[4-(4H-1,2,4-triazol-3-yldiazenyl)phenyl]amino}ethylhydrogensulfat, 2-{Ethyl[4-(4H-1,2,4-triazol-3-yldiazenyl)phenyl]amino}ethylhydrogensulfat, 3-{Ethyl[4-(4H-1,2,4-triazol-3-yldiazenyl)phenyl]amino}propylhydrogensulfat, 3-{Methyl[3-methyl-4-(4H-1,2,4-triazol-3-yldiazenyl)phenyl]amino}propylhydrogensulfat, 3-{Ethyl[3-methyl-4-(4H-1,2,4-triazol-3-yldiazenyl)phenyl]amino}propylhydrogensulfat, 2-{Methyl[4-(1,3,4-thiadiazol-2-yldiazenyl)phenyl]amino}ethylhydrogensulfat, 2-{Ethyl[4-(1,3,4-thiadiazol-2-yldiazenyl)phenyl]amino}ethylhydrogensulfat, 3-{Ethyl[4-(1,3,4-thiadiazol-2-yldiazenyl)phenyl]amino}propylhydrogensulfat, 3-{Methyl[3-methyl-4-(1,3,4-thiadiazol-2-yldiazenyl)phenyl]amino}propylhydrogensulfat, 3-{Ethyl[3-methyl-4-(1,3,4-thiadiazol-2-yldiazenyl)phenyl]amino}propylhydrogensulfat, 2-{Methyl[4-(1,2,4-thiadiazol-5-yldiazenyl)phenyl]amino}ethylhydrogensulfat, 2-{Ethyl[4-(1,2,4-thiadiazol-5-yldiazenyl)phenyl]amino}ethylhydrogensulfat, 3-{Ethyl[4-(1,2,4-thiadiazol-5-yldiazenyl)phenyl]amino}propylhydrogensulfat, 2-{Methyl[3-methyl-4-(1,2,4-thiadiazol-5-yldiazenyl)phenyl]amino}ethylhydrogensulfat, 3-{Ethyl[3-methyl-4-(1,2,4-thiadiazol-5-yldiazenyl)phenyl]amino}propylhydrogensulfat, 2-{Methyl[4-(1,3-benzothiazol-2-yldiazenyl)phenyl]amino}ethylhydrogensulfat, 2-{Ethyl[4-(1,3-benzothiazol-2-yldiazenyl)phenyl]amino}ethylhydrogensulfat, 3-{Ethyl[4-(1,3-benzothiazol-2-yldiazenyl)phenyl]amino}propylhydrogensulfat, 2-{[4-(1,3-Benzothiazol-2-yldiazenyl)-3-methylphenyl](methyl)amino}ethylhydrogensulfat, 3-{Ethyl[4-(1,3-Benzothiazol-2-yldiazenyl)-3-methylphenyl]amino}propylhydrogensulfat, 2-{Methyl[4-(1,3-benzimidazol-2-yldiazenyl)phenyl]amino}ethylhydrogensulfat, 2-{[4-(1,3-Benzimidazol-2-yldiazenyl)phenyl](ethyl)amino}ethylhydrogensulfat, 3-{Ethyl[4-(1,3-benzimidazol-2-yldiazenyl)phenyl]amino}propylhydrogensulfat, 2-{[4-(1,3-Benzimidazol-2-yldiazenyl)-3-methylphenyl](methyl)amino}ethylhydrogensulfat, 3-{[4-(1,3-Benzimidazol-2-yldiazenyl)-3-methylphenyl](ethyl)amino}propylhydrogensulfat, 2-[Methyl({4-[(E)-2-(1,2-oxazol-5-yl)diazen-1-yl]phenyl})amino]ethylhydrogensulfat, 2-[Ethyl({4-[(E)-2-(1,2-oxazol-5-yl)diazen-1-yl]phenyl})amino]ethyl sulfate, 2-[Methyl({2-nitro-4-[2-(1,2-oxazol-5-yl)diazen-1-yl]phenyl})amino]ethylhydrogensulfat, 2-[Ethyl({2-nitro-4-[2-(1,2-oxazol-5-yl)diazen-1-yl]phenyl})amino]ethylhydrogensulfat, 2-[Methyl({3-nitro-4-[2-(1,2-oxazol-5-yl)diazen-1-yl]phenyl})amino]ethylhydrogensulfat, 2-[Ethyl({3-nitro-4-[2-(1,2-oxazol-5-yl)diazen-1-yl]phenyl})amino]ethylhydrogensulfat, 2-[Methyl({4-[2-(4H-1,2,4-triazol-3-yl)diazen-1-yl]phenyl})amino]ethylhydrogensulfat, 2-[Ethyl({4-[2-(4H-1,2,4-triazol-3-yl)diazen-1-yl]phenyl})amino]ethylhydrogensulfat, 2-[Methyl({2-nitro-4-[2-(4H-1,2,4-triazol-3-yl)diazen-1-yl]phenyl})amino]ethylhydrogensulfat, 2-[Ethyl({2-nitro-4-[2-(4H-1,2,4-triazol-3-yl)diazen-1-yl]phenyl})amino]ethylhydrogensulfat, 2-[Methyl({3-nitro-4-[2-(4H-1,2,4-triazol-3-yl)diazen-1-yl]phenyl})amino]ethylhydrogensulfat, 2-[Ethyl({3-[2-(4H-1,2,4-triazol-3-yl)diazen-1-yl]phenyl})amino]ethylhydrogensulfat, 2-[Methyl({4-[2-(4-methyl-4H-1,2,4-triazol-3-yl)diazen-1-yl]-phenyl})amino]ethylhydrogensulfat, 2-[Ethyl({4-[2-(4-methyl-4H-1,2,4-triazol-3-yl)diazen-1-yl]-phenyl})amino]ethylhydrogensulfat, 2-[Methyl({4-[2-(4-methyl-4H-1,2,4-triazol-3-yl)diazen-1-yl]-2-nitrophenyl})amino]ethylhydrogensulfat, 2-[Ethyl({4-[2-(4-methyl-4H-1,2,4-triazol-3-yl)diazen-1-yl]-2-nitrophenyl})amino]ethylhydrogensulfat, 2-[Methyl({4-[2-(4-methyl-4H-1,2,4-triazol-3-yl)diazen-1-yl]-3-nitrophenyl})amino]ethylhydrogensulfat, 2-[Ethyl({4-[2-(4-methyl-4H-1,2,4-triazol-3-yl)diazen-1-yl]-3-nitrophenyl})amino]ethylhydrogensulfat, 2-[Methyl({4-[2-(1-methyl-1H-1,2,4-triazol-3-yl)diazen-1-yl]phenyl})amino]ethylhydrogensulfat, 2-[Ethyl({4-[2-(1-methyl-1H-1,2,4-triazol-3-yl)diazen-1-yl]phenyl})amino]ethylhydrogensulfat, 2-[Methyl({4-[2-(1-methyl-1H-1,2,4-triazol-3-yl)diazen-1-yl]-2-nitrophenyl})amino]ethylhydrogensulfat, 2-[Ethyl({4-[2-(1-methyl-1H-1,2,4-triazol-3-yl)diazen-1-yl]-2-nitrophenyl})amino]ethylhydrogensulfat, 2-[Methyl({4-[2-(1-methyl-1H-1,2,4-triazol-3-yl)diazen-1-yl]-3-nitrophenyl})amino]ethylhydrogensulfat, 2-[Ethyl({4-[2-(1-methyl-1H-1,2,4-triazol-3-yl)diazen-1-yl]-3-nitrophenyl})amino]ethylhydrogensulfat, 2-[Methyl({4-[2-(1,2,4-thiadiazol-5-yl)diazen-1-yl]phenyl})amino]ethylhydrogensulfat, 2-[Ethyl({4-[2-(1,2,4-thiadiazol-5-yl)diazen-1-yl]phenyl})amino]ethylhydrogensulfat, 2-[Methyl({2-nitro-4-[2-(1,2,4-thiadiazol-5-yl)diazen-1-yl]phenyl})amino]ethylhydrogensulfat, 2-[Ethyl({2-nitro-4-[2-(1,2,4-thiadiazol-5-yl)diazen-1-yl]phenyl})amino]ethylhydrogensulfat, 2-[Methyl({3-nitro-4-[2-(1,2,4-thiadiazol-5-yl)diazen-1-yl]phenyl})amino]ethylhydrogensulfat, 2-[Ethyl({3-nitro-4-[2-(1,2,4-thiadiazol-5-yl)diazen-1-yl]phenyl})amino]ethylhydrogensulfat, 2-[Methyl({4-[2-(1,3,4-thiadiazol-2-yl)diazen-1-yl]phenyl})amino]ethylhydrogensulfat, 2-[Ethyl({4-[2-(1,3,4-thiadiazol-2-yl)diazen-1-yl]phenyl})amino]ethylhydrogensulfat, 2-[Methyl({2-nitro-4-[2-(1,3,4-thiadiazol-2-yl)diazen-1-yl]phenyl})amino]ethylhydrogensulfat, 2-[Ethyl({2-nitro-4-[2-(1,3,4-thiadiazol-2-yl)diazen-1-yl]phenyl})amino]ethylhydrogensulfat, 2-[Methyl({3-nitro-4-[2-(1,3,4-thiadiazol-2-yl)diazen-1-yl]phenyl})amino]ethylhydrogensulfat, 2-[Ethyl({3-nitro-4-[2-(1,3,4-thiadiazol-2-yl)diazen-1-yl]phenyl})amino]ethylhydrogensulfat und/oder dem Natrium- oder Kaliumsalz einer der voranstehenden Verbindungen, bevorzugt ausgewählt aus 2-{Methyl[4-(1,3-thiazol-2-yldiazenyl)phenyl]amino}ethylhydrogensulfat, 2-{Ethyl[4-(1,3-thiazol-2-yldiazenyl)phenyl]amino}ethylhydrogensulfat, 3-{Methyl[4-(1,3-thiazol-2-yldiazenyl)phenyl]amino}propylhydrogensulfat, 3-{Ethyl[4-(1,3-thiazol-2-yldiazenyl)phenyl]amino}propylhydrogensulfat, 2-{Methyl[4-(4H-1,2,4-triazol-3-yldiazenyl)phenyl]amino}ethylhydrogensulfat, 2-{Ethyl[4-(4H-1,2,4-triazol-3-yldiazenyl)phenyl]amino}ethylhydrogensulfat, 3-{Ethyl[4-(4H-1,2,4-triazol-3-yldiazenyl)phenyl]amino}propylhydrogensulfat, 2-{Methyl[4-(1,2,4-thiadiazol-5-yldiazenyl)phenyl]amino}ethylhydrogensulfat, 2-{Ethyl[4-(1,2,4-thiadiazol-5-yldiazenyl)phenyl]amino}ethylhydrogensulfat, 3-{Ethyl[4-(1,2,4-thiadiazol-5-yldiazenyl)phenyl]amino}propylhydrogensulfat, 2-{Methyl[4-(1,3-benzothiazol-2-yldiazenyl)phenyl]amino}ethylhydrogensulfat, 2-{Ethyl[4-(1,3-benzothiazol-2-yldiazenyl)phenyl]amino}ethylhydrogensulfat, 3-{Ethyl[4-(1,3-benzothiazol-2-yldiazenyl)phenyl]amino}propylhydrogensulfat, 2-[Methyl({4-[(E)-2-(1,2-oxazol-5-yl)diazen-1-yl]phenyl})amino]ethylhydrogensulfat, 2-[Ethyl({4-[(E)-2-(1,2-oxazol-5-yl)diazen-1-yl]phenyl})amino]ethylhydrogensulfat, 2-[Ethyl({2-nitro-4-[2-(1,2-oxazol-5-yl)diazen-1-yl]phenyl})amino]ethylhydrogensulfat, 2-[Ethyl({3-nitro-4-[2-(1,2-oxazol-5-yl)diazen-1-yl]phenyl})amino]ethylhydrogensulfat, 2-[Ethyl({2-nitro-4-[2-(4H-1,2,4-triazol-3-yl)diazen-1-yl]phenyl})amino]ethylhydrogensulfat, 2-[Ethyl({3-nitro-4-[2-(4H-1,2,4-triazol-3-yl)diazen-1-yl]phenyl})amino]ethylhydrogensulfat, 2-[Methyl({4-[2-(4-methyl-4H-1,2,4-triazol-3-yl)diazen-1-yl]-phenyl})amino]ethylhydrogensulfat, 2-[Ethyl({4-[2-(4-methyl-4H-1,2,4-triazol-3-yl)diazen-1-yl]-phenyl})amino]ethylhydrogensulfat, 2-[Ethyl({4-[2-(4-methyl-4H-1,2,4-triazol-3-yl)diazen-1-yl]-2-nitrophenyl})amino]ethylhydrogensulfat, 2-[Ethyl({4-[2-(4-methyl-4H-1,2,4-triazol-3-yl)diazen-1-yl]-3-nitrophenyl})amino]ethylhydrogensulfat, 2-[Ethyl({4-[2-(4-methyl-4H-1,2,4-triazol-3-yl)diazen-1-yl]-phenyl})amino]ethylhydrogensulfat 2-[Ethyl({4-[2-(1-methyl-1H-1,2,4-triazol-3-yl)diazen-1-yl]phenyl})amino]ethylhydrogensulfat, 2-[Ethyl({4-[2-(1-methyl-1H-1,2,4-triazol-3-yl)diazen-1-yl]-2-nitrophenyl})amino]ethylhydrogensulfat, 2-[Ethyl({4-[2-(1-methyl-1H-1,2,4-triazol-3-yl)diazen-1-yl]-3-nitrophenyl})amino]ethylhydrogensulfat, 2-[Ethyl({2-nitro-4-[2-(1,2,4-thiadiazol-5-yl)diazen-1-yl]phenyl})amino]ethylhydrogensulfat, 2-[Ethyl({3-nitro-4-[2-(1,2,4-thiadiazol-5-yl)diazen-1-yl]phenyl})amino]ethylhydrogensulfat, 2-[Ethyl({2-nitro-4-[2-(1,3,4-thiadiazol-2-yl)diazen-1-yl]phenyl})amino]ethylhydrogensulfat, 2-[Ethyl({3-nitro-4-[2-(1,3,4-thiadiazol-2-yl)diazen-1-yl]phenyl})amino]ethylhydrogensulfat und/oder dem Natrium- oder Kaliumsalz einer der voranstehenden Verbindungen.

9. Mittel nach einem der Ansprüche 1 bis 8, **dadurch gekennzeichnet, dass** es die Verbindung(en) der Formel (I) in einem Anteil von 0,001 bis 5 Gew.-%, vorzugsweise von 0,0025 bis 2,5 Gew.-%, besonders bevorzugt von 0,005 bis 2,0 Gew.-% und insbesondere von 0,01 bis 1,5 Gew.-%, jeweils bezogen auf das Gesamtgewicht des Mittels, enthält.

10. Mittel zum Färben und gleichzeitigen Aufhellen keratinischer Fasern nach einem der Ansprüche 1 bis 9, **dadurch gekennzeichnet, dass** es zusätzlich mindestens ein Oxidationsmittel, ausgewählt aus Natriumpersulfat, Kaliumpersulfat, Ammoniumpersulfat, Wasserstoffperoxid und/oder einem der festen Anlagerungsprodukte von Wasserstoffperoxid an organische oder anorganische Verbindungen und Gemischen der voranstehenden Verbindungen, enthält.

11. Mittel zum Färben und gleichzeitigen Aufhellen keratinischer Fasern nach einem der Ansprüche 1 bis 10, **dadurch gekennzeichnet, dass** es zusätzlich mindestens ein Oxidationsfarbstoffvorprodukt, bevorzugt zumindest ein Oxidationsfarbstoffvorprodukt vom Entwickler-Typ und mindestens ein Oxidationsfarbstoffvorprodukt vom Kuppler-Typ, enthält.

12. Mittel zum Färben und gleichzeitigen Aufhellen keratinischer Fasern nach einem der Ansprüche 1 bis 11, **dadurch gekennzeichnet, dass** es zusätzlich mindestens eine oberflächenaktive Substanz enthält, die ausgewählt ist aus anionischen, kationischen, zwitterionischen, amphoteren und nichtionischen Tensiden und Emulgatoren.

13. Mittel zum Färben und gleichzeitigen Aufhellen keratinischer Fasern nach einem der Ansprüche 1 bis 12, **dadurch gekennzeichnet, dass** der pH-Wert des anwendungsbereiten Mittels, gemessen bei einer Temperatur von 22 °C, zwischen 6 und 11, insbesondere zwischen 7 und 10,5, liegt.

14. Verwendung von Verbindungen der Formel (I) nach einem der Ansprüche 1 bis 8 in Mitteln zur Färbung menschlicher Haare, insbesondere in Mitteln nach Anspruch 1 bis 13,
- zur Verbesserung der Farbintensität und/oder
- zur Verbesserung der Echtheitseigenschaften der Färbung, insbesondere der Waschechtheit, der Lichtechtheit und der Ultrablondierechtheit.

15. Verbindung der Formel (I) gemäß einem der Ansprüche 1 bis 8.

## Claims

1. An agent for dyeing keratin fibers, in particular human hair, **characterized in that** it contains, in a cosmetic carrier, at least one compound of formula (I) 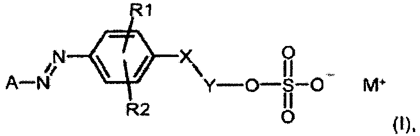 in which
R1, R2, independently of one another, represent hydrogen, a C₁-C₆ alkyl group, a C₂-C₆ alkenyl group, a hydroxy group, a halogen, a C₁-C₆ alkoxy group, an amino group, a nitro group, an acetylamino group or a sulfonamide group, or, provided they are in the ortho-position relative to one another, R1 and R2 form a 5 or 6 member saturated or unsaturated ring that may optionally contain additional heteroatoms,
X represents O or N-R3,
R3 represents hydrogen, a C₁-C₆ alkyl group, a C₂-C₆ alkenyl group, a C₂-C₆ hydroxy alkyl group, a C₂-C₆ polyhydroxy alkyl group, a cyano C₁-C₆ alkyl group or a group -Y'-O-SO₂-O-M⁺,
Y, Y' independently of one another each represent (CH₂)ₙ or C₂H₄-(OC₂H₄)ₙ or (CH₂)ₙ-O-(CH₂)ₘ or (CH₂)ₙ-N(R6')-(CH₂)ₘ, and n and optionally m each represent an integer from 1 to 6,
A represents one of the structures (II) to (XII), (XIV) or (XV) 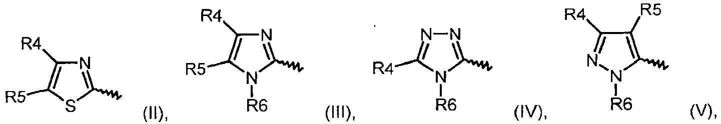 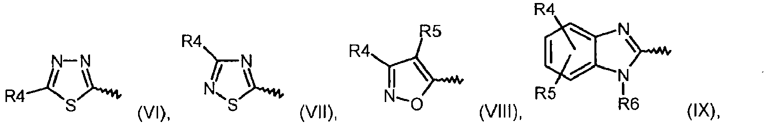 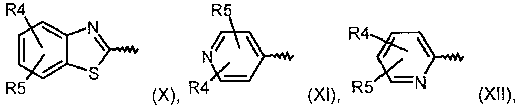 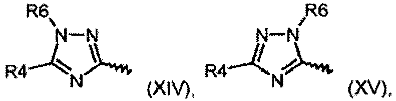
R4, R5 independently of one another each represent hydrogen, an amino group, a C₁-C₆ alkyl amino group, a di-(C₁-C₆-alkyl) amino group, a hydroxy group, a C₁-C₆ alkoxy group, a C₁-C₆ alkyl group, a C₂-C₆ alkenyl group, a carboxylic acid group, a sulfonic acid group, halogen, an acetyl amino group or a sulfonamide group, or, provided they are in the ortho-position relative to one another, R4 and R5 form a 5 or 6 member saturated or unsaturated ring that may optionally contain additional hetero atoms,
R6, R6' independently of one another each represent a hydrogen atom, a C₁-C₆ alkyl group, a C₂-C₆ alkenyl group or a C₂-C₆ hydroxy alkyl group,
and M⁺ represents a proton (H⁺), an alkali metal cation or a half-equivalent of an alkaline earth metal cation.

2. The agent according to claim 1, **characterized in that** it contains at least one compound of formula (I), in which A represents one of the structures (II), (III), (IV), (VI), (VII), (X) or (XIV), in particular (II), (IV) or (X).

3. The agent according to one of claim 1 or claim 2, **characterized in that** it contains at least one compound of formula (I), in which X represents N-R3 and Y represents (CH2)n where n is equal to 2 or 3.

4. The agent according to one of claims 1 to 3, **characterized in that** it contains a compound of formula (I), in which X represents N-R3 and R3 represents a C1-C6 alkyl group, in particular methyl or ethyl.

5. The agent according to one of claims 1 to 4, **characterized in that** it contains a compound of formula (I), in which R1 and R2 independently of one another represent hydrogen, a C1-C6 alkyl group or a nitro group.

6. The agent according to one of claims 1 to 5, **characterized in that** it contains a compound of formula (I), in which R1 and R2 each represent hydrogen.

7. The agent according to one of claims 1 to 5, **characterized in that** it contains a compound of formula (I), in which R1 represents hydrogen and R2 represents a nitro group.

8. The agent according to one of claims 1 to 7, **characterized in that** it contains a compound of formula (I), selected from 2-{methyl[4-(1,3-thiazole-2-yldiazenyl)phenyl]amino}ethyl hydrogen sulfate, 2-{ethyl[4-(1,3-thiazole-2-yldiazenyl)phenyl]amino}ethyl hydrogen sulfate, 3-{methyl[4-(1,3-thiazole-2-yldiazenyl)phenyl]amino}propyl hydrogen sulfate, 3-{ethyl[4-(1,3-thiazole-2-yldiazenyl)phenyl]amino}propyl hydrogen sulfate, 2-{methyl[3-methyl-4-(1,3-thiazole-2-yldiazenyl)phenyl]amino}ethyl hydrogen sulfate, 3-{ethyl[3-methyl-4-(1,3-thiazole-2-yldiazenyl)phenyl]amino}propyl hydrogen sulfate, 2-{[4-(1H-imidazole-2-yldiazenyl)phenyl](methyl)amino}ethyl hydrogen sulfate, 2-{ethyl[4-(1H-imidazole-2-yldiazenyl)phenyl]amino}ethyl hydrogen sulfate, 3-{ethyl[4-(1H-imidazole-2-yldiazenyl)phenyl]amino}propyl hydrogen sulfate, 2-{ethyl[4-(1H-imidazole-2-yldiazenyl)-3-methylphenyl]amino}ethyl hydrogen sulfate, 3-{ethyl[4-(1H-imidazole-2-yldiazenyl)-3-methylphenyl]amino}propyl hydrogen sulfate, 2-{methyl[4-(4H-1,2,4-triazole-3-yldiazenyl)phenyl]amino}ethyl hydrogen sulfate, 2-{ethyl[4-(4H-1,2,4-triazole-3-yldiazenyl)phenyl]amino}ethyl hydrogen sulfate, 3-{ethyl[4-(4H-1,2,4-triazole-3-yldiazenyl)phenyl]amino}propyl hydrogen sulfate, 3-{methyl[3-methyl-4-(4H-1,2,4-triazole-3-yldiazenyl)phenyl]amino}propyl hydrogen sulfate, 3-{ethyl[3-methyl-4-(4H-1,2,4-triazole-3-yldiazenyl)phenyl]amino}propyl hydrogen sulfate, 2-{methyl[4-(1,3,4-thiadiazole-2-yldiazenyl)phenyl]amino}ethyl hydrogen sulfate, 2-{ethyl[4-(1,3,4-thiadiazole-2-yldiazenyl)phenyl]amino}ethyl hydrogen sulfate, 3-{ethyl[4-(1,3,4-thiadiazole-2-yldiazenyl)phenyl]amino}propyl hydrogen sulfate, 3-{methyl[3-methyl-4-(1,3,4-thiadiazole-2-yldiazenyl)phenyl]amino}propyl hydrogen sulfate, 3-{ethyl[3-methyl-4-(1,3,4-thiadiazole-2-yldiazenyl)phenyl]amino}propyl hydrogen sulfate, 2-{methyl[4-(1,2,4-thiadiazole-5-yldiazenyl)phenyl]amino}ethyl hydrogen sulfate, 2-{ethyl[4-(1,2,4-thiadiazole-5-yldiazenyl)phenyl]amino}ethyl hydrogen sulfate, 3-{ethyl[4-(1,2,4-thiadiazole-5-yldiazenyl)phenyl]amino}propyl hydrogen sulfate, 2-{methyl[3-methyl-4-(1,2,4-thiadiazote-5-yldiazenyl)phenyl]amino}ethyl hydrogen sulfate, 3-{ethyl[3-methyl-4-(1,2,4-thiadiazole-5-yldiazenyl)phenyl]amino}propyl hydrogen sulfate, 2-{methyl[4-(1,3-benzothiazole-2-yldiazenyl)phenyl]amino}ethyl hydrogen sulfate, 2-{ethyl[4-(1,3-benzothiazole-2-yldiazenyl)phenyl]amino}ethyl hydrogen sulfate, 3-{ethyl[4-(1,3-benzothiazole-2-yldiazenyl)phenyl]amino}propyl hydrogen sulfate, 2-{[4-(1,3-benzothiazole-2-yldiazenyl)-3-methylphenyl](methyl)amino}ethyl hydrogen sulfate, 3-{ethyl[4-(1,3-benzothiazole-2-yldiazenyl)-3-methyl phenyl]amino}propyl hydrogen sulfate, 2-{methyl[4-(1,3-benzimidazole-2-yldiazenyl)phenyl]amino}ethyl hydrogen sulfate, 2-{[4-(1,3-benzimidazole-2-yldiazenyl)phenyl](ethyl)amino}ethyl hydrogen sulfate, 3-{ethyl[4-(1,3-benzimidazole-2-yldiazenyl)phenyl]amino}propyl hydrogen sulfate, 2-{[4-(1,3-benzimidazole-2-yldiazenyl)-3-methylphenyl](methyl)amino}ethyl hydrogen sulfate, 3-{[4-(1,3-benzimidazole-2-yldiazenyl)-3-methylphenyl](ethyl)amino}propyl hydrogen sulfate, 2-[methyl({4-[(E)-2-(1,2-oxazole-5-yl)diazene-1-yl]phenyl})amino]ethyl hydrogen sulfate, 2-[ethyl({4-[(E)-2-(1,2-oxazole-5-yl)diazene-1-yl]phenyl})amino]ethyl sulfate, 2-[methyl({2-nitro-4-[2-(1,2-oxazole-5-yl)diazene-1-yl]phenyl})amino]ethyl hydrogen sulfate, 2-[ethyl({2-nitro-4-[2-(1,2-oxazole-5-yl)diazene-1-yl]phenyl})amino]ethyl hydrogen sulfate, 2-[methyl({3-nitro-4-[2-(1,2-oxazole-5-yl)diazene-1-yl]phenyl})amino]ethyl hydrogen sulfate, 2-[ethyl({3-nitro-4-[2-(1,2-oxazole-5-yl)diazene-1-yl]phenyl})amino]ethyl hydrogen sulfate, 2-[methyl({4-[2-(4H-1,2,4-triazole-3-yl)diazene-1-yl]phenyl})amino]ethyl hydrogen sulfate, 2-[ethyl({4-[2-(4H-1,2,4-triazole-3-yl)diazene-1-yl]phenyl})amino] ethyl hydrogen sulfate, 2-[methyl({2-nitro-4-[2-(4H-1,2,4-triazole-3-yl)diazene-1-yl]phenyl})amino]ethyl hydrogen sulfate, 2-[ethyl({2-nitro-4-[2-(4H-1,2,4-triazole-3-yl)diazene-1-yl]phenyl})amino]ethyl hydrogen sulfate, 2-[methyl({3-nitro-4-[2-(4H-1,2,4-triazole-3-yl)diazene-1-yl]phenyl})amino]ethyl hydrogen sulfate, 2-[ethyl({3-[2-(4H-1 ,2,4-triazole-3-yl)diazene-1-yl]phenyl})amino]ethyl hydrogen sulfate, 2-[methyl({4-[2-(4-methyl-4H-1,2,4-triazole-3-yl)diazene-1-yl]-phenyl})amino]ethyl hydrogen sulfate, 2-[ethyl({4-[2-(4-methyl-4H-1,2,4-triazole-3-yl)diazene-1-yl]-phenyl})amino]ethyl hydrogen sulfate, 2-[methyl({4-[2-(4-methyl-4H-1,2,4-triazole-3-yl)diazene-1-yl]-2-nitrophenyl})amino]ethyl hydrogen sulfate, 2-[ethyl({4-[2-(4-methyl-4H-1,2,4-triazole-3-yl)diazene-1-yl]-2-nitrophenyl})amino]ethyl hydrogen sulfate, 2-[methyl({4-[2-(4-methyl-4H-1,2,4-triazole-3-yl)diazene-1-yl]-3-nitrophenyl})amino]ethyl hydrogen sulfate, 2-[ethyl({4-[2-(4-methyl-4H-1,2,4-triazole-3-yl)diazene-1-yl]-3-nitrophenyl})amino]ethyl hydrogen sulfate, 2-[methyl({4-[2-(1-methyl-1H-1,2,4-triazole-3-yl)diazene-1-yl]-phenyl})amino]ethyl hydrogen sulfate, 2-[ethyl({4-[2-(1-methyl-1H-1,2,4-triazole-3-yl)diazene-1-yl]-phenyl})amino]ethyl hydrogen sulfate, 2-[methyl({4-[2-(1-methyl-1H-1,2,4-triazole-3-yl)diazene-1-yl]-2-nitrophenyl})amino]ethyl hydrogen sulfate, 2-[ethyl({4-[2-(1-methyl-1H-1,2,4-triazole-3-yl)diazene-1-yl]-2-nitrophenyl})amino]ethyl hydrogen sulfate, 2-[methyl({4-[2-(1-methyl-1H-1,2,4-triazole-3-yl)diazene-1-yl]-3-nitrophenyl})amino]ethyl hydrogen sulfate, 2-[ethyl({4-[2-(1-methyl-1H-1,2,4-triazole-3-yl)diazene-1-yl]-3-nitrophenyl})amino]ethyl hydrogen sulfate, 2-[methyl({4-[2-(1,2,4-thiadiazole-5-yl)diazene-1-yl]phenyl})amino]ethyl hydrogen sulfate, 2-[ethyl({4-[2-(1,2,4-thiadiazole-5-yl)diazene-1-yl]phenyl})amino]ethyl hydrogen sulfate, 2-[methyl({2-nitro-4-[2-(1,2,4-thiadiazol-5-yl)diazene-1-yl]phenyl})amino]ethyl hydrogen sulfate, 2-[ethyl({2-nitro-4-[2-(1,2,4-thiadiazole-5-yl)diazene-1-yl]phenyl})amino]ethyl hydrogen sulfate, 2-[methyl({3-nitro-4-[2-(1,2,4-thiadiazole-5-yl)diazene-1-yl]phenyl})amino]ethyl hydrogen sulfate, 2-[ethyl({3-nitro-4-[2-(1,2,4-thiadiazole-5-yl)diazene-1-yl]phenyl})amino]ethyl hydrogen sulfate, 2-[methyl({4-[2-(1,3,4-thiadiazole-2-yl)diazene-1-yl]phenyl})amino]ethyl hydrogen sulfate, 2-[ethyl({4-[2-(1,3,4-thiadiazole-2-yl)diazene-1-yl]phenyl})amino]ethyl hydrogen sulfate, 2-[methyl({2-nitro-4-[2-(1,3,4-thiadiazole-2-yl)diazene-1-yl]phenyl})amino]ethyl hydrogen sulfate, 2-[ethyl({2-nitro-4-[2-(1,3,4-thiadiazole-2-yl)diazene-1-yl]phenyl})amino]ethyl hydrogen sulfate, 2-[methyl({3-nitro-4-[2-(1,3,4-thiadiazole-2-yl)diazene-1-yl]phenyl})amino]ethyl hydrogen sulfate, 2-[ethyl({3-nitro-4-[2-(1,3,4-thiadiazole-2-yl)diazene-1-yl]phenyl})amino]ethyl hydrogen sulfate,
and/or the sodium or potassium salt of one of the preceding compounds, preferably selected from
2-{methyl[4-(1,3-thiazole-2-yldiazenyl)phenyl]amino}ethyl hydrogen sulfate, 2-{ethyl[4-(1,3-thiazole-2-yldiazenyl)phenyl]amino}ethyl hydrogen sulfate, 3-{methyl[4-(1,3-thiazole-2-yldiazenyl)phenyl]amino}propyl hydrogen sulfate, 3-{ethyl[4-(1,3-thiazole-2-yldiazenyl)phenyl]amino}propyl hydrogen sulfate, 2-{methyl[4-(4H-1,2,4-triazole-3-yldiazenyl)phenyl]amino}ethyl hydrogen sulfate, 2-{ethyl[4-(4H-1,2,4-triazole-3-yldiazenyl)phenyl]amino}ethyl hydrogen sulfate, 3-{ethyl[4-(4H-1,2,4-triazole-3-yldiazenyl)phenyl]amino}propyl hydrogen sulfate, 2-{methyl[4-(1,2,4-thiadiazole-5-yldiazenyl)phenyl]amino}ethyl hydrogen sulfate, 2-{ethyl[4-(1,2,4-thiadiazole-5-yldiazenyl)phenyl]amino}ethyl hydrogen sulfate, 3-{ethyl[4-(1,2,4-thiadiazole-5-yldiazenyl)phenyl]amino}propyl hydrogen sulfate, 2-{methyl[4-(1,3-benzothiazole-2-yldiazenyl)phenyl]amino}ethyl hydrogen sulfate, 2-{ethyl[4-(1,3-benzothiazole-2-yldiazenyl)phenyl]amino}ethyl hydrogen sulfate, 3-{ethyl[4-(1,3-benzothiazole-2-yldiazenyl)phenyl]amino}propyl hydrogen sulfate, 2-[methyl({4-[(E)-2-(1,2-oxazole-5-yl)diazene-1-yl]phenyl})amino]ethyl hydrogen sulfate, 2-[ethyl({4-[(E)-2-(1,2-oxazole-5-yl)diazene-1-yl]phenyl})amino]ethyl hydrogen sulfate, 2-[ethyl({2-nitro-4-[2-(1,2-oxazole-5-yl)diazene-1-yl]phenyl})amino]ethyl hydrogen sulfate, 2-[ethyl({3-nitro-4-[2-(1,2-oxazole-5-yl)diazene-1-yl]phenyl})amino]ethyl hydrogen sulfate, 2-[ethyl({2-nitro-4-[2-(4H-1,2,4-triazole-3-yl)diazene-1-yl]phenyl})amino]ethyl hydrogen sulfate, 2-[ethyl({3-nitro-4-[2-(4H-1,2,4-triazole-3-yl)diazene-1-yl]phenyl})amino]ethyl hydrogen sulfate, 2-[methyl({4-[2-(4-methyl-4H-1,2,4-triazole-3-yl)diazene-1-yl]-phenyl})amino]ethyl hydrogen sulfate, 2-[ethyl({4-[2-(4-methyl-4H-1,2,4-triazole-3-yl)diazene-1-yl]-phenyl})amino]ethyl hydrogen sulfate 2-[ethyl({4-[2-(4-methyl-4H-1,2,4-triazole-3-yl)diazene-1-yl]-2-nitrophenyl})amino]ethyl hydrogen sulfate, 2-[ethyl({4[2-(4-methyl-4H-1,2,4-triazole-3-yl)diazene-1-yl]-3-nitrophenyl})amino]ethyl hydrogen sulfate, 2-[ethyl({4-[2-(4-methyl-4H-1,2,4-triazole-3-yl)diazene-1-yl)-phenyl})amino]ethyl hydrogen sulfate, 2-[ethyl({4-[2-(1-methyl-1H-1,2,4-triazole-3-yl)diazene-1-yl]-phenyl})amino]ethyl hydrogen sulfate 2-[ethyl({4-[2-(1-methyl-1H-1,2,4-triazole-3-yl)diazene-1-yl]-2-nitrophenyl})amino]ethyl hydrogen sulfate, 2-[ethyl({4[2-(1-methyl-1H-1,2,4-triazole-3-yl)diazene-1-yl]-3-nitrophenyl})amino]ethyl hydrogen sulfate, 2-[ethyl({2-nitro-4-[2-(1,2,4-thiadiazole-5-yl)diazene-1-yl]phenyl})amino]ethyl hydrogen sulfate, 2-[ethyl({3-nitro-4-[2-(1,2,4-thiadiazole-5-yl)diazene-1-yl]phenyl})amino]ethyl hydrogen sulfate, 2-[ethyl({2-nitro-4-[2-(1,3,4-thiadiazole-2-yl)diazene-1-yl]phenyl})amino]ethyl hydrogen sulfate, 2-[ethyl({3-nitro-4-[2-(1,3,4-thiadiazole-2-yl)diazene-1-yl]phenyl})amino]ethyl hydrogen sulfate
and/or the sodium or potassium salt of one of the preceding compounds.

9. The agent according to one of claims 1 to 8, **characterized in that** it contains the compound(s) of formula (I) in a proportion of from 0.001 to 5 wt.%, preferably from 0.0025 to 2.5 wt.%, more preferably from 0.005 to 2.0 wt.%, and in particular from 0.01 to 1.5 wt.%, in each case based on the total weight of the agent.

10. The agent for dyeing and simultaneously lightening keratin fibers according to one of claims 1 to 9, **characterized in that** it additionally contains at least one oxidizing agent selected from sodium persulfate, potassium persulfate, ammonium persulfate, hydrogen peroxide and/or one of the solid addition products of hydrogen peroxide to organic or inorganic compounds and mixtures of the preceding compounds.

11. The agent for dyeing and simultaneously lightening keratin fibers according to one of claims 1 to 10, **characterized in that** it additionally contains at least one oxidation dye precursor, preferably at least one oxidation dye precursor of the developer type and at least one oxidation dye precursor of the coupler type.

12. The agent for dyeing and simultaneously lightening keratin fibers according to one of claims 1 to 11, **characterized in that** it additionally contains at least one surface-active substance that is selected from anionic, cationic, zwitterionic, amphoteric and non-ionic surfactants and emulsifiers.

13. The agent for dyeing and simultaneously lightening keratin fibers according to one of claims 1 to 12, **characterized in that** the pH of the ready-to-use agent, measured at a temperature of 22°C, is between 6 and 11, in particular between 7 and 10.5.

14. Use of compounds of formula (I) according to one of claims 1 to 8 in agents for dyeing human hair, in particular in agents according to claims 1 to 13,
- for improving the color intensity and/or
- for improving the fastness properties of the coloring, in particular the wash fastness, the light fastness and the ultra-bleaching fastness.

15. A compound of formula (I) according to any of claims 1 to 8.

## Revendications

1. Produit de coloration de fibres de kératine, en particulier de cheveux humains, **caractérisé en ce qu'**il contient, dans un vecteur cosmétique, au moins un composé de formule (I) : 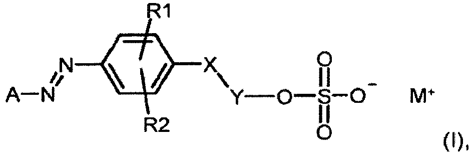 dans lequel :
R1, R2, représentent indépendamment l'un de l'autre un hydrogène, un groupe C₁-₆-alkyle, un groupe C₂₋₆-alcényle, un groupe hydroxy, un halogène, un groupe C₁₋₆-alcoxy, un groupe amino, un groupe nitro, un groupe acétylamino ou un groupe sulfonamide, ou R1 et R2 forment, s'ils se trouvent en position ortho l'un par rapport à l'autre, un cycle saturé ou insaturé à 5 ou 6 chaînons, pouvant éventuellement contenir d'autres hétéroatomes,
X représente O ou N-R3,
R3 représente un hydrogène, un groupe C₁₋₆-alkyle, un groupe C₂₋₆-alcényle, un groupe un groupe C₂₋₆-hydroxyalkyle, un groupe C₂₋₆-polyhydroxyalkyle, un groupe cyano-C₁₋₆-alkyle ou un groupe -Y'-O-SO₂-O- M⁺,
Y, Y' représentent respectivement, indépendamment l'un de l'autre, -(CH₂)ₙ- ou - C₂H₄-(OC₂H₄)ₙ- ou -(CH₂)ₙ-O-(CH₂)ₘ- ou -(CH₂)ₙ-N(R6')-(CH₂)ₘ-, où n et éventuellement m représentent respectivement un entier entre 1 et 6,
A représente une des structures (II) à (XIV) ou (XV) : 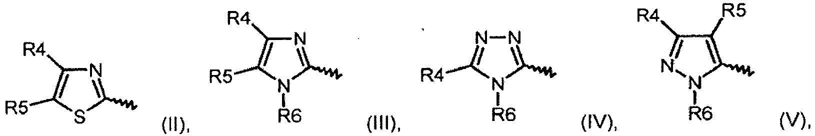 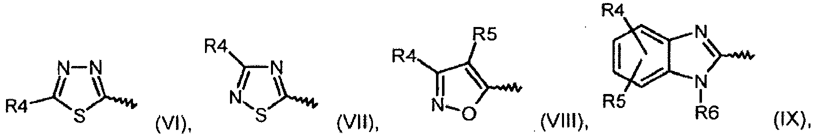 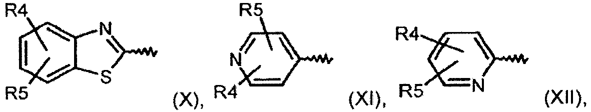 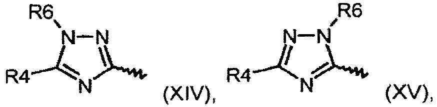
R4, R5 représentent respectivement, indépendamment l'un de l'autre, un hydrogène, un groupe amino, un groupe C₁₋₆-alkylamino, un groupe di-(C₁-₆-alkyl)amino, un groupe hydroxy, un groupe C₁₋₆-alcoxy, un groupe C₁₋₆-alkyle, un groupe C₂₋₆-alcényle, un groupe acide carboxylique, un groupe acide sulfonique, un halogène, un groupe acétylamino ou un groupe sulfonamide, ou R4 et R5 forment, s'ils se trouvent en position ortho l'un par rapport à l'autre, un cycle saturé ou insaturé à 5 ou 6 chaînons, pouvant éventuellement contenir d'autres hétéroatomes,
R6, R6' représentent respectivement, indépendamment l'un de l'autre, un hydrogène, un groupe C₁₋₆-alkyle, un groupe C₂₋₆-alcényle ou un groupe C₂₋₆-hydroxyalkyle, et
M⁺ représente un proton (H⁺), un cation de métal alcalin ou demi-équivalent d'un cation de métal alcalinoterreux.

2. Produit selon la revendication 1, **caractérisé en ce qu'**il contient au moins un composé de formule (I), dans lequel A représente une des structures (II), (III), (IV), (VI), (VII), (X) ou (XIV), en particulier (II), (IV) ou (X).

3. Produit selon une des revendications 1 ou 2, **caractérisé en ce qu'**il contient au moins un composé de formule (I), dans lequel X représente N-R3 et Y représente -(CH₂)ₙ avec n = 2 ou 3.

4. Produit selon une des revendications 1 à 3, caractérisé en qu'il contient au moins un composé de formule (I), dans lequel X représente N-R3 et R3 représente un groupe C₁₋₆-alkyle, en particulier méthyle ou éthyle.

5. Produit selon une des revendications 1 à 4, caractérisé en qu'il contient au moins un composé de formule (I), dans lequel R1 et R2 représentent, indépendamment l'un de l'autre, un hydrogène, un groupe C₁₋₆-alkyle ou un groupe nitro.

6. Produit selon une des revendications 1 à 5, caractérisé en qu'il contient au moins un composé de formule (I), dans lequel R1 et R2 représentent respectivement un hydrogène.

7. Produit selon une des revendications 1 à 5, caractérisé en qu'il contient au moins un composé de formule (I), dans lequel R1 représente un hydrogène et R2 représente un groupe nitro.

8. Produit selon une des revendications 1 à 7, caractérisé en qu'il contient au moins un composé de formule (I) choisi parmi : 2-{méthyl-[4-(1,3-thiazol-2-yldiazényl)phényl]amino}éthylhydrogénosulfate 2-{éthyl-[4-(1,3-thiazol-2-yldiazényl)phényl]amino}éthylhydrogénosulfate 3-{méthyl-[4-(1,3-thiazol-2-yldiazényl)phényl]amino}propylhydrogénosulfate 3-{éthyl-[4-(1,3-thiazol-2-yldiazényl)phényl]amino}propylhydrogénosulfate 2-{méthyl-[3-méthyl-4-(1,3-thiazol-2-yldiazényl)phényl]amino}éthylhydrogénosulfate 3-{éthyl-[3-méthyl-4-(1,3-thiazol-2-yldiazényl)phényl]amino}propylhydrogénosulfate 2-{[4-(1H-imidazol-2-yldiazényl)phényl](méthyl)amino}éthylhydrogénosulfate 2-{éthyl-[4-(1H-imidazol-2-yldiazényl)phényl]amino}éthylhydrogénosulfate 3-{éthyl-[4-(1H-imidazol-2-yldiazényl)phényl]amino}propylhydrogénosulfate 2-{éthyl-[4-(1H-imidazol-2-yldiazényl)-3-méthylphényl]amino}éthylhydrogénosulfate 3-{éthyl-[4-(1H-imidazol-2-yldiazényl)-3-méthylphényl]amino}propylhydrogénosulfate 2-{méthyl-[4-(4H-1,2,4-triazol-3-yldiazényl)phényl]amino}éthylhydrogénosulfate 2-{éthyl-[4-(4H-1,2,4-triazol-3-yldiazényl)phényl]amino}éthylhydrogénosulfate 3-{éthyl-[4-(4H-1,2,4-triazol-3-yldiazényl)phényl]amino}propylhydrogénosulfate 3-{méthyl-[3-méthyl-4-(4H-1,2,4-triazol-3-yl-diazényl)phényl]amino}propylhydrogénosulfate 3-{éthyl-[3-méthyl-4-(4H-1,2,4-triazol-3-yl-diazényl)phényl]amino}propylhydrogénosulfate 2-{méthyl-[4-(1,3,4-thiadazol-2-yldiazényl)phényl]amino}éthylhydrogénosulfate 2-{éthyl-[4-(1,3,4-thiadazol-2-yldiazényl)phényl]amino}éthylhydrogénosulfate 3-{méthyl-[4-(1,3,4-thiadazol-2-yldiazényl)phényl]amino}propylhydrogénosulfate 3-{méthyl-[3-méthyl-4-(1,3,4-thiadazol-2-yl-diazényl)phényl]amino}propylhydrogénosulfate 3-{éthyl-[3-méthyl-4-(1,3,4-thiadazol-2-yl-diazényl)phényl]amino}propylhydrogénosulfate 2-{méthyl-[4-(1,2,4-thiadazol-5-yldiazényl)phényl]amino}éthylhydrogénosulfate 2-{éthyl-[4-(1,2,4-thiadazol-5-yldiazényl)phényl]amino}éthylhydrogénosulfate 3-{éthyl-[4-(1,2,4-thiadazol-5-yldiazényl)phényl]amino}propylhydrogénosulfate 2-{méthyl-[3-méthyl-4-(1,2,4-thiadazol-5-yl-diazényl)phényl]amino}éthylhydrogénosulfate 3-{méthyl-[3-méthyl-4-(1,2,4-thiadazol-5-yl-diazényl)phényl]amino}propylhydrogénosulfate 2-{méthyl-[4-(1,3-benzothiazol-2-yldiazényl)phényl]amino}éthylhydrogénosulfate 2-{éthyl-[4-(1, 3-benzothiazol-2-yldiazényl)phényl]amino}éthylhydrogénosulfate 3-{éthyl-[4-(1,3-benzothiazol-2-yldiazényl)phényl]amino}propylhydrogénosulfate 2-{[4-(1,3-benzothiazol-2-yldiazényl)-3-méthylphényl](méthyl)amino}éthylhydrogénosulfate 3-{éthyl-[4-(1,3-benzothiazol-2-yldiazényl)-3-méthylphényl]amino}propylhydrogénosulfate 2-{méthyl-[4-(1,3-benzimidazol-2-yldiazényl)phényl]amino}éthylhydrogénosulfate 2-{[4-(1,3-benzimidazol-2-yldiazényl)phényl]amino}éthylhydrogénosulfate 3-{éthyl-[4-(1,3-benzimidazol-2-yldiazényl)phényl]amino}propylhydrogénosulfate 2-{[4-(1,3-benzimidazol-2-yldiazényl)-3-méthylphényl](méthyl)amino}éthylhydrogénosulfate 3-{[4-(1,3-benzimidazol-2-yldiazényl)-3-méthylphényl](éthyl)amino}propylhydrogénosulfate 2-[méthyl-({4-[(E)-2-(1,2-oxazol-5-yt)diazén-1-yl]phényl})amino]éthylhydrogénosulfate 2-[éthyl-({4-[(E)-2-(1,2-oxazol-5-yl)diazén-1-yl]phényl})amino]éthylsulfate 2-[méthyl-({2-nitro-4-[2-(1,2-oxazol-5-yl)diazén-1-yl]phényl})amino]éthylhydrogénosulfate 2-[éthyl-({2-nitro-4-[2-(1,2-oxazol-5-yl)diazén-1-yl]phényl})amino]éthylhydrogénosulfate 2-[méthyl-({3-nitro-4-[2-(1,2-oxazol-5-yl)diazén-1-yl]phényl})amino]éthylhydrogénosulfate 2-[éthyl-({3-nitro-4-[2-(1,2-oxazol-5-yl)diazén-1-yl]phényl})amino]éthylhydrogénosulfate 2-[méthyl-({4-[2-(4H-1,2,4-triazol-3-yl)diazén-1-yl]phényl})amino]éthylhydrogénosulfate 2-[éthyl-({4-[2-(4H-1,2,4-triazol-3-yl)diazén-1-yl]phényl}amino]éthylhydrogénosulfate 2-[méthyl-({2-nitro-4-[2-(4H-1,2,4-triazol-3-yl)diazén-1-yl]phényl})amino]éthylhydrogénosulfate 2-[éthyl-({2-nitro-4-[2-(4H-1,2,4-triazol-3-yl)diazén-1-yl]phényl})amino]éthylhydrogénosulfate 2-[méthyl-({3-nitro-4-[2-(4H-1,2,4-triazol-3-yl)diazén-1-yl]phényl})amino]éthylhydrogénosulfate 2-[éthyl-({3-(2-(4H-1,2,4-triazol-3-yl)diazén-1-yl]phényl})amino]éthylhydrogénosulfate 2-[méthyl-({4-[2-(4-méthyl-4H-1,2,4-triazol-3-yl)diazén-1-yl]phényl})amino]éthylhydrogénosulfate 2-[éthyl-({4-[2-(4-méthyl-4H-1,2,4-triazol-3-yl)diazén-1-yl]phényl})amino]éthylhydrogénosulfate 2-[méthyl-({4-[2-(4-méthyl-4H-1,2,4-triazol-3-yl)diazén-1-yl]-2-nitrophényl})amino]éthylhydrogéno sulfate 2-[éthyl-({4-[2-(4-méthyl-4H-1,2,4-triazol-3-yl)diazén-1-yl]-2-nitrophényl})amino]éthylhydrogéno sulfate 2-[méthyl-({4-[2-(4-méthyl-4H-1,2,4-triazol-3-yl)diazén-1-yl]-3-nitrophényl})amino]éthylhydrogéno sulfate 2-[éthyl-({4-[2-(4-méthyl-4H-1,2,4-triazol-3-yl)diazén-1-yl]-3-nitrophényl})amino]éthylhydrogéno sulfate 2-[méthyl-({4-[2-(1-méthyl-1H-1,2,4-triazol-3-yl)diazén-1-yl]phényl})amino]éthylhydrogénosulfate 2-[méthyl-({4-[2-(1-méthyl-1H-1,2,4-triazol-3-yl)diazén-1-yl]phényl})amino]éthylhydrogénosulfate 2-[méthyl-({4-[2-(1-méthyl-1H-1,2,4-triazol-3-yl)diazén-1-yl]phényl})amino]éthylhydrogénosulfate 2-[éthyl-({4-[2-(1-méthyl-1H-1,2,4-triazol-3-yl)diazén-1-yl]-2-phényl})amino]éthylhydrogénosulfate 2-[méthyl-({4-[2-(1-méthyl-1H-1,2,4-triazol-3-yl)diazén-1-yl]-2-nitrophényl})amino]éthylhydrogéno sulfate 2-[éthyl-({4-[2-(1-méthyl-4H-1,2,4-triazol-3-yl)diazén-1-yl]-2-nitrophényl})amino]éthylhydrogéno sulfate 2-[méthyl-({4-[2-(1,2,4-thiadiazol-3-yl)diazén-1-yl]-3-nitrophényl})amino]éthylhydrogénosulfate 2-[éthyl-({4-[2-(1,2,4-thiadiazol-3-yl)diazén-1-yl]-3-nitrophényl})amino]éthylhydrogénosulfate 2-[méthyl-({4-[2-(1,2,4-thiadiazol-5-yl)diazén-1-yl]phényl})amino]éthylhydrogénosulfate 2-[éthyl-({4-[2-(1,2,4-thiadiazol-5-yl)diazén-1-yl]phényl})amino]éthylhydrogénosulfate 2-[méthyl-({2-nitro-4-[2-(1,2,4-thiadiazôl-5-yl)diazén-1-yl]phényl})amino]éthylhydrogénosulfate 2-[éthyl-({2-nitro-4-[2-(1,2,4-thiadiazol-5-yl)diazén-1-yl]phényl})amino]éthylhydrogénosulfate 2-[méthyl-({3-nitro-4-[2-(1,2,4-thiadiazol-5-yl)diazén-1-yl]phényl})amino]éthylhydrogénosulfate 2-[éthyl-({3-nitro-4-[2-(1,2,4-thiadiazol-5-yl)diazén-1-yl]phényl})amino]éthylhydrogénosulfate 2-[méthyl-({4-[2-(1,3,4-thiadiazol-2-yl)diazén-1-yl]phényl})amino]éthylhydrogénosulfate 2-[éthyl-({4-[2-(1,3,4-thiadiazol-2-yl)diazén-1-yl]phényl})amino]éthylhydrogénosulfate 2-[méthyl-({2-nitro-4-[2-(1,3,4-thiadiazol-2-yl)diazén-1-yl]phényl})amino]éthylhydrogénosulfate 2-[éthyl-({2-nitro-4-[2-(1,3,4-thiadiazol-2-yl)diazén-1-yl]phényl})amino]éthylhydrogénosulfate 2-[méthyl-({3-nitro-4-[2-(1,3,4-thiadiazol-2-yl)diazén-1-yf]phényl})amino]éthylhydrogénosulfate 2-[éthyl-({3-nitro-4-[2-(1,3,4-thiadiazol-2-yl)diazén-1-yl]phényl})amino]éthylhydrogénosulfate
et/ou le sel de sodium ou de potassium d'un des composés ci-dessus, préférentiellement choisi parmi :
2-{méthyl-[4-(1,3-thiazol-2-yldiazényl)phényl]amino}éthylhydrogénosulfate
2-{éthyl-[4-(1,3-thiazol-2-yldiazényl)phényl]amino}éthylhydrogénosulfate
3-{méthyl-[4-(1,3-thiazol-2-yldiazényl)phényl]amino}propylhydrogénosulfate
3-{éthyl-[4-(1,3-thiazol-2-yldiazényl)phényl]amino}propylhydrogénosulfate
2-{méthyl-[4-(4H-1,2,4-triazol-3-yldiazényl)phényl]amino}éthylhydrogénosulfate
2-{éthyl-[4-(4H-1,2,4-triazol-3-yldiazényl)phényl]amino}éthylhydrogénosulfate
3-{éthyl-[4-(4H-1,2,4-triazol-3-yldiazényl)phényl]amino}propylhydrogénosulfate
2-{méthyl-[4-(1,2,4-thiadazol-5-yldiazényl)phényl]amino}éthylhydrogénosulfate
2-{éthyl-[4-(1,2,4-thiadazol-5-yldiazényl)phényl]amino}éthylhydrogénosulfate
3-{éthyl-[4-(1,2,4-thiadazol-5-yldiazényl)phényl]amino}propylhydrogénosulfate
2-{méthyl-[4-(1,3-benzothiazol-2-yldiazényl)phényl]amino}éthylhydrogénosulfate
2-{éthyl-[4-(1,3-benzothiazol-2-yldiazényl)phényl]amino}éthylhydrogénosulfate
3-{éthyl-[4-(1,3-benzothiazol-2-yldiazényl)phényl]amino}propylhydrogénosulfate
2-[méthyl-({4-[(E)-2-(1,2-oxazol-5-yl)diazén-1-yl]phényl})amino]éthylhydrogénosulfate
2-[éthyl-({4-[(E)-2-(1,2-oxazol-5-yl)diazén-1-yl]phényl})amino]éthylsulfate
2-[éthyl-({2-nitro-4-[2-(1,2-oxazol-5-yl)diazén-1-yl]phényl})amino]éthylhydrogénosulfate
2-[éthyl-({3-nitro-4-[2-(1,2-oxazol-5-yl)diazén-1-yl]phényl})amino]éthylhydrogénosulfate
2-[éthyl-({2-nitro-4-[2-(4H-1,2,4-triazol-3-yl)diazén-1-yl]phényl})amino]éthylhydrogénosulfate
2-[éthyl-({3-nitro-4-[2-(4H-1,2,4-triazol-3-yl)diazén-1-yl]phényl})amino]éthylhydrogénosulfate
2-[méthyl-({4-[2-(4-méthyl-4H-1,2,4-triazol-3-yl)diazén-1-yl]phényl})amino]éthylhydrogénosulfate
2-[éthyl-({4-[2-(4-méthyl-4H-1,2,4-triazol-3-yl)diazén-1-yl]phényl})amino]éthylhydrogénosulfate
2-[éthyl-({4-[2-(4-méthyl-4H-1,2,4-triazol-3-yl)diazén-1-yl]-2-nitrophényl})amino]éthylhydrogénosulfate
2-[éthyl-({4-[2-(4-méthyl-4H-1,2,4-triazol-3-yl)diazén-1-yl]-3-nitrophényl})amino]éthylhydrogénosulfate
2-[éthyl-({4-[2-(4-méthyl-4H-1,2,4-triazol-3-yl)diazén-1-yl]phényl})amino]éthylhydrogénosulfate
2-[éthyl-({4-[2-(1-méthyl-1H-1,2,4-triazol-3-yl)diazén-1-yl]phényl})amino]éthylhydrogénosulfate
2-[éthyl-({4-[2-(1-méthyl-1H-1,2,4-triazol-3-yl)diazén-1-yl]-2-nitrophényl})amino]éthylhydrogénosulfate
2-[éthyl-({4-[2-(1-méthyl-1H-1,2,4-triazol-3-yl)diazén-1-yl]-3-nitrophényl})amino]éthylhydrogéno sulfate
2-[éthyl-({2-nitro-4-[2-(1,2,4-thiadiazol-5-yl)diazén-1-yl]phényl})amino]éthylhydrogénosulfate
2-[éthyl-({3-nitro-4-[2-(1,2,4-thiadiazol-5-yl)diazén-1-yl]phényl})amino]éthylhydrogénosulfate
2-[éthyl-({2-nitro-4-[2-(1,2,4-thiadiazol-2-yl)diazén-1-yl]phényl})amino]éthylhydrogénosulfate
2-[éthyl-({3-nitro-4-[2-(1,2,4-thiadiazol-2-yl)diazén-1-yl]phényl})amino]éthylhydrogénosulfate
et/ou le sel de sodium ou de potassium d'un des composés ci-dessus.

9. Produit selon une des revendications 1 à 8, caractérisé en qu'il contient le ou les composés de formule (I) à hauteur de 0,001 à 5 % en poids, de préférence de 0,0025 à 2,5 % en poids, particulièrement préférentiellement de 0,005 à 2,0 % en poids et en particulier de 0,01 à 1,5 % en poids, respectivement rapportés au poids total du produit.

10. Produit de coloration et en même temps d'éclaircissement de fibres de kératine selon une des revendications 1 à 9, **caractérisé en ce qu'**il contient en plus au moins un oxydant choisi parmi le persulfate de sodium, le persulfate de potassium, le persulfate d'ammonium, le peroxyde d'hydrogène et/ou un produit d'addition solide du peroxyde d'hydrogène à des composés organiques ou minéraux, et leurs mélanges.

11. Produit de coloration et en même temps d'éclaircissement de fibres de kératine selon une des revendications 1 à 10, **caractérisé en ce qu'**il contient en plus un précurseur de colorant d'oxydation, de préférence a moins un précurseur de colorant d'oxydation de type développeur et au moins un précurseur de colorant d'oxydation de type coupleur.

12. Produit de coloration et en même temps d'éclaircissement de fibres de kératine selon une des revendications 1 à 11, **caractérisé en ce qu'**il contient en plus au moins un tensioactif choisi parmi des tensioactif et émulsifiants anioniques, cationiques, zwitterioniques, amphotères et non-ioniques.

13. Produit de coloration et en même temps d'éclaircissement de fibres de kératine selon une des revendications 1 à 12, **caractérisé en ce que** le pH du produit prêt à l'emploi, mesuré à une température de 22°C, est entre 6 et 11, en particulier entre 7 et 10,5.

14. Utilisation de composés de formule (I) selon une des revendications 1 à 8 dans des produits de coloration de cheveux humains, en particulier de produits selon une des revendications 1 à 13 :
- pour améliorer l'intensité de la coloration, et/ou
- pour améliorer les propriétés de résistance de la coloration, en particulier de résistance au lavage, à la lumière et suréclaircissement.

15. Composé de formule (I) selon une des revendications 1 à 8.
